(19) Europäisches Patentamt
European Patent Office
Office européen des brevets

(11) **EP 4 552 690 A2**

(12) **EUROPEAN PATENT APPLICATION**

(43) Date of publication:
**14.05.2025 Bulletin 2025/20**

(21) Application number: **25153983.9**

(22) Date of filing: **13.11.2020**

(51) International Patent Classification (IPC):
**A61P 9/10** *(2006.01)*

(52) Cooperative Patent Classification (CPC):
**A61K 35/34; A61P 9/04; A61P 9/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.11.2019 US 201962936189 P**

(62) Document number(s) of the earlier application(s) in accordance with Art. 76 EPC:
**20829738.2 / 4 058 036**

(71) Applicants:
• **Secretome Therapeutics, Inc.**
  **Dallas, TX 75247 (US)**
• **University of Maryland, Baltimore**
  **Baltimore, MD 21201 (US)**

(72) Inventors:
• **MISHRA, Rachana**
  **Baltimore, 21201 (US)**

• **KARATHANASIS, Sotirios K.**
  **Baltimore, 21201 (US)**
• **KAUSHAL, Sunjay**
  **Baltimore, 21201 (US)**
• **SHARMA, Sudhish**
  **Baltimore, 21201 (US)**

(74) Representative: **Brand Murray Fuller LLP**
  **50 Eastcastle Street**
  **London W1W 8EA (GB)**

Remarks:
•This application was filed on 25.01.2025 as a divisional application to the application mentioned under INID code 62.
•Claims filed after the date of filing of the application (Rule 68(4) EPC).

(54) **IMMORTALIZED CARDIAC STEM CELLS FOR CARDIAC REPAIR**

(57) A pharmaceutical composition, comprising IGF-1 and one or more paracrine factor(s) selected from the group consisting of Ang-1, HGF, SDF-1, VEGF-A, bFGF, PDGF-B, and SCF14; wherein the pharmaceutical composition is a solution comprising a conditioned medium

500μl          1ml          1ml          1ml

A: 1.0ml          B: 4.5ml          C: 4.0ml          D: 4.0ml          E: 4.0ml

10,000Cells/ml   1000Cells/ml   200Cells/ml   40Cells/ml   8Cells/ml

Figure 1

**Description**

**TECHNICAL FIELD**

The field of the disclosure concerns at least the fields of cell biology, molecular biology, and medicine, including cardiology.

BACKGROUND

Heart disease in adults is a leading cause of death and there is an increasing number of children with heart failure owing to advances in surgical techniques for congenital heart defects and post-operative ICU care (Go et al., 2014; Go et al., 2014). Two of the important biological processes known to contribute to a wide range of medical conditions, including many of the underlying causes of heart disease, include inflammation and fibrosis. For centuries it was thought that, in contrast to other tissues like the liver and skin, the heart was a terminally differentiated organ unable to regenerate (reviewed in Buja 2019). This paradigm was overturned recently when it was shown that cardiomyocytes in the adult human heart turn over and are replaced at a small but detectable rate of about 1-2% per year (reviewed in Vujic et al 2019). This motivated academics and pharmaceutical companies alike to identify and target basic mechanisms of cardiac regeneration for the treatment of various heart diseases. There are two possible mechanisms for cardiac regeneration: 1) cardiomyocyte replication and 2) the presence of endogenous cardiac stem cells able to proliferate and differentiate into cardiomyocytes. It is now clear that cardiomyocyte proliferation, although it occurs in specialized circumstances such as in amphibians, certain fishes, and neonatal mammals, does not contribute to cardiac regeneration in adult mammals. In contrast, it has been recently shown in clinical studies that transplantation of resident cardiac stem cells can repair/regenerate/remodel human myocardium, leading to improvements in cardiac function as indicated by improvements in ejection fraction, reduced scar size, reduced end diastolic and systolic volumes and improvements in quality of life and NYHA class (Garbern et al., 2013).

A population of cardiac stem cells expressing the cell surface marker c-kit (also known as CD117) was described nearly fifteen years ago and homogenous, healthy populations of c-kit+ cells have been shown to provide anti-inflammatory and anti-fibrotic properties. Enrichment of these cells by magnetic selection of c-kit expressing cardiac cells followed by cardiac delivery of the cells in animal models of cardiac disease, such as myocardial infarction, indicated consistent improvements in cardiac function. Similar promising results were also observed in early clinical testing of these cells. However, with continuous scientific investigation of these cells, two issues became abundantly clear. First, these c-kit+ cardiac stem cells are a mixture of many different progenitor cells, most of which (~90%) are hematopoietic and endothelial cells, not cardiogenic stem cells (Vicinanza et al. 2017). Second, most of the cardiac c-kit+ cells obtained from adult human hearts are senescent, contributing very little to cardiac repair, and possibly even contributing to cardiac damage via the various inflammatory factors secreted by such senescent cells (Lewis-McDouggal et al. 2019).

Accordingly, there is a need in the art for compositions and methods for treating cardiac medical conditions, such as heart failure caused by damaged myocardial tissue, and for addressing the inflammatory and fibrotic processes observed in a wide range of medical conditions. The present invention meets these needs and offers other related advantages.

BRIEF SUMMARY

Embodiments of the disclosure concern methods and compositions related to particular human cardiac stem cells (hCSCs), especially neonatal cardiac stem cells (nCSCs), immortalized nCSCs (Im-nCSCs) (e.g., clonal isolates) and conditioned media produced by Im-nCSCs, for use in the treatment of medical conditions. In particular embodiments, the medical condition is a cardiac medical condition. Although any inflammatory, fibrotic or cardiac medical condition may be treated with such compositions and methods, in specific embodiments the condition is a cardiac condition that would benefit from repairing, regenerating or remodeling cardiac muscle (myocardium). In certain embodiments, the methods and compositions facilitate or enhance the reparative, regenerative or remodeling capacity of nCSCs. In certain embodiments, the medical condition is an inflammatory condition or disease. In more specific embodiments, the inflammatory condition or disease is selected from, but not limited to, ischemic stroke, acute and chronic kidney disease, arthritic conditions, dermatological conditions and COVID-19. In still other embodiments, the anti-fibrotic characteristics of the present invention can improve wound healing and conditions characterized by chronic fibrosis.

Embodiments of the disclosure encompass methods and compositions concerning immortalized cells that originate from the myocardium of a mammal, such as a human, particularly a neonatal human. In certain specific embodiments, the cells comprise immortalized neonatal CD117+ cardiac stem cells, especially immortalized clonal isolates of neonatal CD117+ cardiac stem cells.

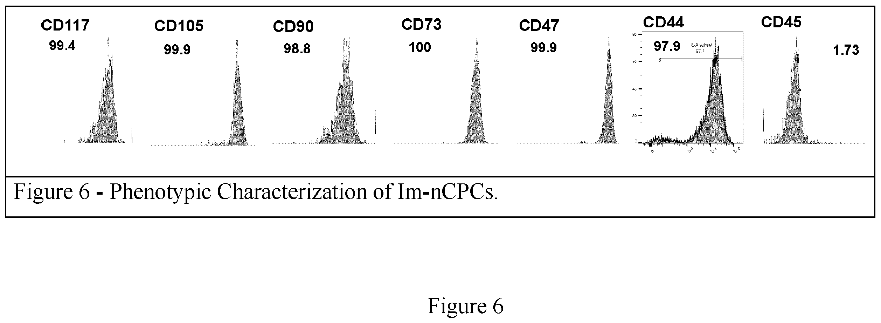

In further embodiments, the immortalized stem cells of the invention have one or more, two or more, three or more, four or more, or five or more, of the following cell surface marker characteristics: CD90$^+$, CD105$^+$, CD117$^+$, CD44$^+$, CD73$^+$, CD47$^+$ CD31$^-$, CD34$^-$, CD45$^-$ and tryptase negative (e.g., Figure 6).

In other embodiments, the immortalized stem cells of the invention can be reversibly immortalized, optionally with a kill switch.

In some embodiments, immortalized cardiac stem cells are provided to an individual for treatment of one or more cardiac medical conditions. In other embodiments a conditioned medium from immortalized cardiac stem cells are provided to an individual for treatment of a cardiac medical condition. In still other embodiments, embodiments, secretomes from the cells are provided to an individual for treatment of a cardiac medical condition. In still other embodiments, one or more immortalized cell clonal isolate-derived trophic factors are provided to an individual for treatment of a cardiac medical condition. In certain other embodiments, any combination of the above may be used in the treatment of a cardiac or other medical condition.

In some embodiments, the present disclosure provides a composition, comprising a conditioned medium (CM) from one or more immortalized neonatal cardiac stem cells (Im-nCSCs), such as a CD117+ immortalized neonatal cardiac stem cell. In certain specific embodiments, the immortalized neonatal cardiac stem cells are an immortalized clonal isolate. In other embodiments, the immortalized neonatal cardiac stem cells have one or more, two or more, three or more, four or more, or five or more, of the following characteristics CD90$^+$, CD105$^+$, CD117$^+$, CD44$^+$, CD73$^+$, CD47$^+$ CD31$^-$, CD34$^-$, CD45$^-$, and tryptase negative. In still other embodiments, the immortalized neonatal cardiac stem cells have all of the following characteristics: CD90$^+$, CD105$^+$, CD117$^+$, CD44$^+$, CD73$^+$, CD47$^+$ CD31$^-$, CD34$^-$, CD45$^-$, and tryptase negative. In still other embodiments, the immortalized neonatal cardiac stem cells have one or more, two or more, three or more or four or more of the following characteristics: GATA4-, CD44+, tryptase negative, CD80-, CD86-. In further embodiments, the immortalized neonatal cardiac stem cells have all of the following characteristics: GATA4-, CD44+, tryptase negative, CD80-, CD86-. In still further embodiments, the immortalized neonatal cardiac stem cells have at one or more, two or more, or all of the following characteristics: CD117+, CD45- and Lin$^-$. In still further embodiments, the immortalized neonatal cardiac stem cells have the following characteristics: CD117$^+$ and CD45$^-$.

In some embodiments of the invention, prior to immortalization, the neonatal cardiac stem cells are isolated from the heart of a neonatal individual (e.g., from a biopsy from the individual). In certain related embodiments, the stem cells are isolated by single cell cloning. In still other embodiments, the stem cells are isolated by single cell cloning not involving any cell selection step as is typically performed in the literature. In some embodiments, the individual from whom the cells are derived is less than 30 days old when the cells are obtained from the individual's heart (e.g., when a biopsy was taken). In some embodiments, the isolation does not comprise contacting the cells with an antibody for cell selection. In some embodiments, the isolation does not comprise a cell enrichment step using antibody-based selection. In some embodiments, the cells are isolated by limited dilution culture. In some embodiments, the immortalization is achieved by the exogenous expression of the human telomerase (hTERT) gene, for example using a delivery vector, such as a lentiviral expression vector.

In other embodiments of the invention, there are provided compositions comprising a plurality of immortalized human neonatal cardiac stem cell clonal isolates.

In still other embodiments, the present disclosure provides a method of treating an individual, e.g., for a cardiac medical condition or other condition described herein, comprising the step of providing to the individual a therapeutically effective amount of a composition comprising a conditioned medium from immortalized neonatal cardiac stem cells. In some embodiments, the composition is selected from any one of the compositions provided herein.

In some embodiments, the present disclosure provides a method of treating an individual for a cardiac medical condition, comprising the step of providing to the individual a therapeutically effective amount of a composition comprising a conditioned medium from immortalized neonatal cardiac stem cells in combination with a composition comprising neonatal cardiac stem cells, such as immortalized neonatal cardiac stem cells. In some embodiments, the composition comprising the conditioned medium is selected from any one of the compositions provided herein.

In still other embodiments, the present disclosure provides a method for isolating a cell clones from immortalized neonatal cardiac stem cells, the method comprising isolating or having isolated one or more cells from a neonatal cardiac tissue and culturing the one or more cells in a suitable culture medium to promote proliferation. In more specific embodiments, the isolating step does not comprise contacting the cells with a moiety that binds a specific cell surface protein such as CD117.

In additional embodiments, the isolated cell clonal isolates immortalized according to the present disclosure have one or more, two or more, three or more, four or more, or five or more, of the following characteristics when they are isolated: GATA4-, CD44+, CD47+ CD31-, CD34-, CD45-, tryptase-, CD80-, CD86- and the immortalized cells maintained such expression patterns following immortalization.

In further embodiments, immortalized cells of the disclosure are originally obtained from the heart of a neonatal individual, prior to immortalization, including a pediatric or non-pediatric individual. In specific embodiments, the individual has a cardiac medical condition. In certain embodiments, the individual has normal myocardium. The cells may be from the myocardium of a pediatric individual with end stage heart failure, in some embodiments. The myocardium may come from a neonatal individual with congenital heart disease.

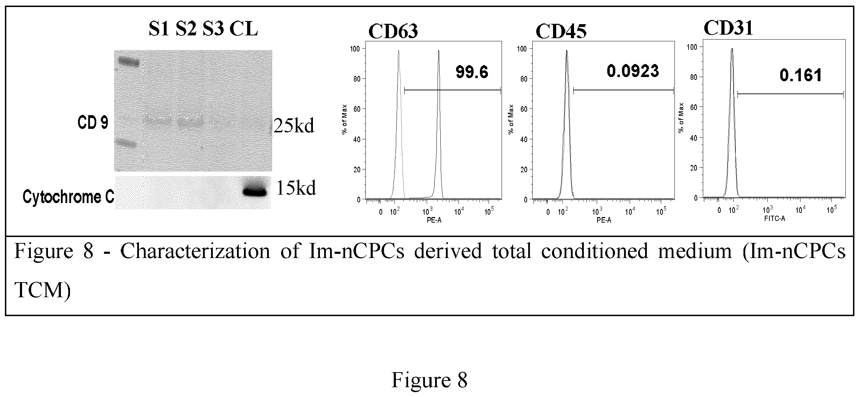

In other specific embodiments, the immortalized cells of the present disclosure secrete exosomes that are CD63$^+$, CD73$^+$, CD47$^+$, CD45$^-$, CD31$^-$ (e.g., Figure 8). The immortalized cells secrete pro-angiogenic and angiogenic cytokines; like VEGF-A, HGF, SCF, SDF-1$\alpha$, IGF, PDGF-B and ANG-1 (e.g., Table 1).

In still other embodiments, there are provided methods of treating an individual for a medical condition, such as a cardiac medical condition or any other condition described herein, comprising the step of providing to the individual a therapeutically effective amount of a composition of the disclosure comprising, for example, immortalized neonatal cardiac stem cells, medium conditioned by immortalized neonatal cardiac stem cells, exosomes secreted by immortalized neonatal cardiac stem cells and/or any combination thereof. In more particular embodiments, the composition comprises independently secreted proteins and/or exosomes from immortalized neonatal cardiac stem cells.

In other specific embodiments a composition of the invention is used to treat a cardiac medical condition, such as by delivering the composition by intramyocardial injection, intravenous injection, cell encapsulation, e.g., in devices that retain the cells but allow secretion of paracrine factors produced by the cells into the circulation, or the like. In still other specific embodiments, the cardiac medical condition is heart failure, cardiomyopathy or congenital heart disease.

In additional embodiments of the invention, there are provided compositions comprising total conditioned medium (TCM) from immortalized cells of the disclosure and/or components thereof as well as methods of using the same in therapeutic amounts for the treatment of a medical condition described herein. In still further embodiments, there are provided compositions comprising exosomes from the immortalized cells of the disclosure and/or components thereof, as well as methods of using the same in in therapeutic amounts for the treatment of a medical condition described herein.

## BRIEF DESCRIPTION OF THE DRAWINGS

**Figure 1** shows illustrative methodology of cell dilution of nCSCs for initiating single cell cultures.

**Figure 2** shows representative phase contrast images of single cell cultures in 96 well plates initiated with cells diluted by the method illustrated in Figure 1.

**Figure 3** shows the phenotypic characterization by flow cytometry analysis of seven clonal isolates of nCSCs obtained using the methodologies shown in Figures 1 and 2.

**Figure 4** shows an illustrative lentiviral vector carrying the hTERT gene used for immortalizing nCSCs isolated via the methods disclosed herein.

**Figure 5** shows representative phase contrast microscopic images of nCSCs and Im-nCSCs..

**Figure 6** shows results from flow cytometry analysis of Im-nCSCs proliferated from single cell cultures at passage 16.

**Figure 7** shows the cardiac functional improvement after myocardial infarction in rat hearts. Left ventricular ejection fraction (EF) and fractional shortening were analyzed by echocardiography.

**Figure 8** shows results from the characterization of Im-nCSCs derived total conditioned medium (Im-nCSCs TCM)

**Figure 9** shows that Im-nCSCs TCM protects from hydrogen peroxide induced apoptosis in neonatal rat cardiomyocytes

**Figure 10** shows that Im-nCSCs TCM promotes angiogenesis.

**Figure 11** shows that Im-nCSCs TCM promotes cell migration and in-vitro wound healing.

**Figure 12** shows the in-vivo functional activity of nCSCs and Im-nCSCs TCM in the rat myocardial infarction model by intravenous injection, measured by echocardiogram.

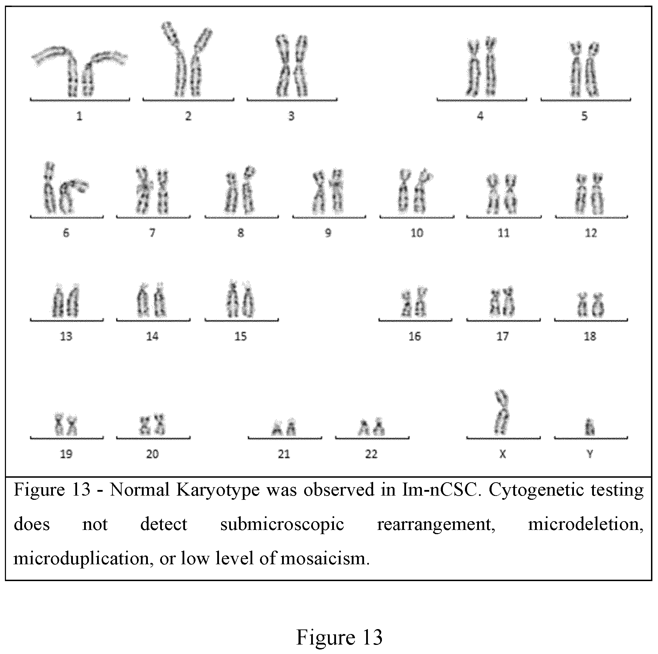

**Figure 13** shows a representative normal karyotype observed in Im-nCSCs.

## DETAILED DESCRIPTION

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (*i.e.,* to at least one) of the grammatical object of the article. By way of example, "an element" means one element or more than one element.

The term "and/or" is used in this disclosure to mean either "and" or "or" unless indicated otherwise.

The term "e.g." is used herein to mean "for example," and will be understood to imply the inclusion of a stated step or element or group of steps or elements but not the exclusion of any other step or element or group of steps or elements.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

The term "administering", as used herein, refers to any mode of transferring, delivering, introducing, or transporting matter such as a compound, e.g. a pharmaceutical compound, or other agent such as an antigen, to a subject. Modes of administration include oral administration, topical contact, intravenous, intraperitoneal, intramuscular, intranasal, or subcutaneous administration. Administration "in combination with" further matter such as one or more therapeutic agents includes simultaneous (concurrent) and consecutive administration in any order.

Throughout this specification, unless the context requires otherwise, the words "comprise," "comprises," and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

An "effective amount," when used in connection with a compound, is an amount of the compound needed to elicit a desired response. In some embodiments, the desired response is a biological response, e.g., in a subject. In some embodiments, the compound may be administered to a subject in an effective amount to effect a biological response in the subject. In some embodiments, the effective amount is a "therapeutically effective amount."

The terms "therapeutically effective amount" and "therapeutic dose" are used interchangeably herein to refer to an amount of a composition (e.g., a conditioned medium from a neonatal CSC such as an immortalized CSC disclosed herein), which is effective following administration to a subject for treating a disease or disorder in the subject as described herein.

The term "modulating" includes "increasing," "enhancing" or "stimulating," as well as "decreasing" or "reducing," typically in a statistically significant or a physiologically significant amount as compared to a control. An "increased," "stimulated" or "enhanced" amount is typically a "statistically significant" amount, and may include an increase that is 1.1, 1.2, 2, 3, 4, 5, 6, 7, 8, 9, 10, 15, 20, 30 or more times (e.g., 500, 1000 times) (including all integers and decimal points in between and above 1, e.g., 1.5, 1.6, 1.7. 1.8, etc.) the amount produced by no composition or a control composition, sample or test subject. A "decreased" or "reduced" amount is typically a "statistically significant" amount, and may include a 1%, 2%, 3%, 4%, 5%, 6%, 7%, 8%, 9%, 10%, 11%, 12%, 13%, 14%, 15%, 16%, 17%, 18% , 19%, 20%, 25%, 30%, 35%, 40%, 45%, 50%, 55%, 60%, 65%, 70%, 75%, 80%, 85%, 90%, 95%, or 100% decrease in the amount produced by no composition (the absence of an agent or compound) or a control composition, including all integers in between.

A "subject," or "patient" as used herein, includes any animal that exhibits a symptom, or is at risk for exhibiting a symptom, which can be treated with a composition disclosed herein (e.g., a conditioned medium from a neonatal CSC such as an immortalized hCSC disclosed herein). Suitable subjects (patients) include human patients. Suitable subjects also include laboratory animals (such as mouse, rat, rabbit, or guinea pig), farm animals (such as pig, horse, cow), and domestic animals or pets (such as a cat or dog). Non-human primates (such as a monkey, chimpanzee, baboon or rhesus) are also included.

"Substantially" or "essentially" means nearly totally or completely, for instance, 95% or greater of some given quantity.

"Treatment" or "treating," as used herein, includes any desirable effect on the symptoms or pathology of a disease or condition, and may include even minimal changes or improvements in one or more measurable markers of the disease or condition being treated. "Treatment" or "treating" does not necessarily indicate complete eradication or cure of the disease or condition, or associated symptoms thereof. The subject receiving this treatment is any subject in need thereof. Exemplary markers of clinical improvement will be apparent to persons skilled in the art.

The terms "CD117" and "c-kit" can be used interchangeably in accordance with the present disclosure in reference to the same protein that goes synonymously by both names. The c-kit/CD117 protein, as well as the gene which encodes it, have been extensively characterized and is well known in the art (e.g., https://www.uniprot.org/uniprot/P10721 and UNIPROT accession number P10721).

Unless defined otherwise, all technical and scientific terms used herein have the same meaning as commonly understood by one of ordinary skill in the art to which the invention belongs. Although any methods, compositions, reagents, cells, similar or equivalent to those described herein can be used in the practice or testing of the invention, the preferred methods and materials are described herein. All publications and references, including but not limited to patents and patent applications, cited in this specification are herein incorporated by reference in their entirety as if each individual publication or reference were specifically and individually indicated to be incorporated by reference herein as being fully set forth. Any patent application to which this application claims priority is also incorporated by reference herein in its entirety in the manner described above for publications and references.

The term "cardiac stem cells" as used herein may be defined as cells derived from cardiac tissue and are clonogenic, multipotent, and self-renewing. In specific embodiments, these cardiac stem cells express one or more of the following: CD117, CD90, CD105, CD73, CD44, and CD47 and are negative for one or more of the following: CD31, C34, CD45, and tryptase.

**Overview**

The present disclosure provides, inter alia, compositions and methods related to human neonatal cardiac stem cells (nCSCs) and methods of isolating and immortalizing the same, as well as conditioned medium harvested from cultures of such cardiac stem cells, and methods of using such cells, medium and/or cells resuspended in conditioned medium therapeutically, e.g., to treat, repair, regenerate and/or remodel cardiac tissues and treat medical conditions caused by inflammatory and/or fibrotic processes (e.g., a cardiac medical condition).

**I. Cells of the Disclosure and Conditioned Media and Secretomes Thereof**

Embodiments of the disclosure concern mammalian cells, or conditioned medium from the cells, that provide a therapeutic function for an individual in need thereof. In specific embodiments, the cells are immortalized human neonatal cardiac stem cells (Im-nCSCs) useful for achieving a therapeutic function or effect in a mammalian heart. In some embodiments, the cells are immortalized human neonatal cardiac stem cells, Im-nCSCs, and they secrete one or more agents that are anti-inflammatory, anti-fibrotic, pro-angiogenic and/or useful for a therapeutic function or effect in a mammalian heart. In specific embodiments, the cells are immortalized human neonatal heart CSCs that have the activity of reparative, regenerative or remodeling capacity themselves upon administration to a heart *in vivo* and/or have the activity for facilitating reparative, regenerative or remodeling capacity of endogenous cells and/or tissue in a human myocardium upon administration to a heart *in vivo.* In specific embodiments, the cells are immortalized neonatal human CSCs and they secrete one or more proteins that are anti-inflammatory, anti-fibrotic, pro-angiogenic, and/or have the activity of reparative, regenerative or remodeling capacity upon administration to a heart *in vivo* and/or have the activity for facilitating reparative, regenerative or remodeling capacity of endogenous cells and/or tissue in a human myocardium upon administration to a heart *in vivo.* In specific embodiments, the cells secrete one or more proteins that are capable of eliciting a bystander effect, such that delivery of a conditioned media derived from the cells to the myocardium *in vivo,* enhances repair, regeneration or remodeling of endogenous cells and/or tissues.

In specific embodiments, the immortalized human neonatal heart CSCs are made by obtaining human neonatal heart CSCs clonal isolates and immortalizing them by any suitable immortalization method. Many such methods are known in the art are suitable for use in the present invention including, for example, and without limitation, immortalization by introduction of the Simian virus 40 large T-antigen (Kobayashi et al., (2000) Science 287: 1258-62, Nakamura et al., (1997) Transplantation 63 (11): 1541-47), transfection of antisense constructions against p53 and retinoblastoma protein (Werner et al., (2000) Biotechnol Bioeng 68 (1): 59-70), transgenic introduction of a truncated Met protein (Amicone et al., (1997) EMBO J. 16 (3):495 503), and expression of a hepatitis C virus core protein (Ray et al., (2000) Virology 271: 197-204).

In one particular embodiment, the human neonatal heart CSCs of the present disclosure are immortalized by stably transfecting them with a vector (e.g., a lentivirus vector) expressing human telomerase (hTERT). The mechanism restricting the *in vitro* proliferation of human fibroblast cells has been shown to be the progressive shortening of telomeres with each cell division (Hayflicket al., (1961) Exp. Cell Res. 25:585-621). Telomeres constitute the terminal regions of chromosomes, and shortened telomeres trigger the restriction of proliferation. Stem cells, however, are able to avoid telomere-dependent proliferative restriction by adding telomeric repeat sequences onto chromosome ends using telomerase reverse transcriptase (Greider et al., (1985) Cell 43:405-413). The ability to achieve telomerase reconstitution of human neonatal heart CSCs to develop stable neonatal human cardiac-derived clonal stem cell lines having the phenotypic characteristics of neonatal cardiac stem cells after passage in vitro for use, e.g., in cardiac-directed cell therapies and cardiac studies, ensures cell consistency and eliminates the need of multiple donors for producing large quantities of these cells for therapeutic uses. For example, in some embodiments, such cells may serve as a cell factory capable of producing unlimited quantities of conditioned media comprising one or more secreted factors, e.g., secreted proteins, that have the activity of reparative, regenerative or remodeling capacity upon administration to a heart in vivo and/or have the activity for facilitating reparative, regenerative or remodeling capacity of endogenous cells and/or tissue in a human myocardium upon administration to a heart in vivo. In other embodiments, such cells may serve as a cell factory capable of producing unlimited quantities of conditioned media comprising one or more secreted factors, e.g., secreted proteins that have anti-inflammatory, anti-fibrotic and pro-angiogenic properties that can provide therapeutic benefits in wound healing and/or medical conditions beyond heart diseases.

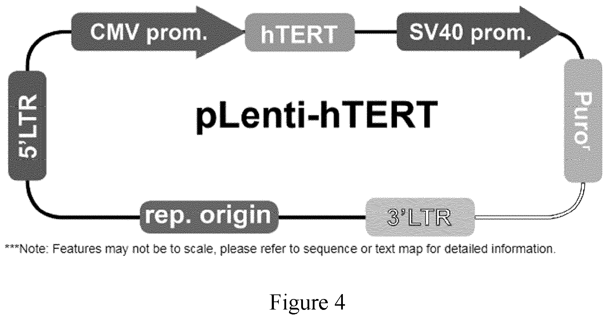

The telomerase used for immortalization may be encoded by a human TERT (hTERT) gene, for example. The human neonatal heart CSCs may be transfected with hTERT using any suitable methodology. For example, the human neonatal heart CSCs may be infected with a recombinant virus capable of transferring the hTERT gene into the cell. In another example, the human neonatal heart CSCs may be infected with a lentiviral viral vector and individual CSC clones containing hTERT may be isolated and expanded. The lentiviral vector may comprise hTERT under the control of a suitable promoter (e.g., the CMV promoter), an illustrative example of which is shown in Figure 4.

In one aspect, the invention provides a population of immortalized human cells that express a human telomerase, wherein the population exhibits phenotypic features of neonatal human cardiac stem cells at early passage and continues to express said phenotypic features at late passage (e.g., very large population doubling levels (PDLs) in the range of 70 or more) in vitro. In another aspect, the invention provides an immortalized human cell that expresses a human telomerase, wherein the cell exhibits phenotypic features of a neonatal human cardiac stem cells at early passage in vitro and continues to express said phenotypic features at late passage in vitro. In one embodiment, the immortalized cells can be induced to differentiate into the major types of cardiac cells (i.e. endothelial, smooth muscle, cardiomyocytes) in-vitro.

In additional embodiments of the invention, the immortalized cells may be used to produce conditioned media. In certain embodiments, the conditioned media may be used in any of a variety of indications, e.g., to treat cardiac and other medical conditions, to induce angiogenesis, to inhibit inflammation, to promote cardiomyocyte salvage and to reduce cardiac fibrosis in vivo. (Ongstad et al. 2019)

In some embodiments, the immortalized cell expresses CD117. In other embodiments, the immortalized cell expresses CD117 at a high level. In various embodiments, reference herein to "high levels" of CD117 expression means that >80% of cells analyzed (e.g., by flow cytometry) express CD117. In still other embodiments, reference herein to "low levels" of CD117 expression means that <80% of cells analyzed (e.g., by flow cytometry) express CD117.

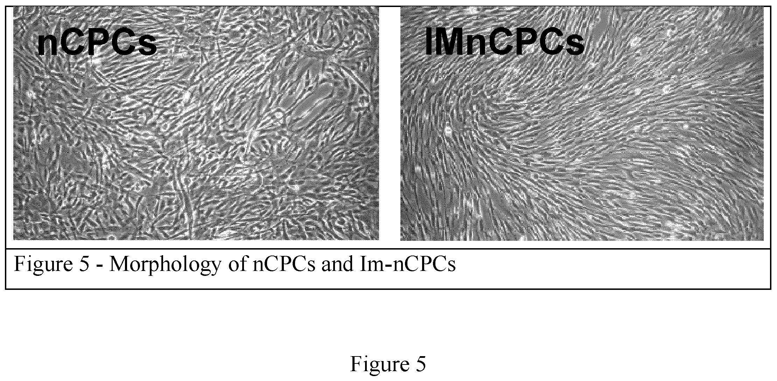

In certain embodiments, the morphology of the cell before and after immortalization is not substantially changed and/or is characterized by displaying no evidence of senescence (Figure 5).

In other embodiments, the immortalized cell does not express CD31. In one embodiment, the immortalized cell does not express CD45. In still other embodiments, the immortalized cell expresses CD117, but does not express CD31 or CD45. In other particular embodiments, the immortalized cell expresses CD117 at a high level but does not express CD31 or CD45 (Figure 6). In other particular embodiments, the immortalized cell express CD117, CD90, CD105, CD44, CD47 and CD73 but does not express CD31 or CD45 (Figure 6).

The neonatal cardiac stem cell may be obtained from any suitable source prior to immortalization. In particular embodiments, the source of the cardiac stem cells that are immortalized are from a neonatal individual or from an individual in utero. In particular embodiments, the cells are not adult cardiac stem cells. The cells may be obtained from an individual in need of therapeutic use using progeny of the same cells or the cells may be obtained from another individual. The cells may originate from a donated heart of a neonatal individual or from the heart of a living neonatal individual. The cells may be provided commercially to the individual in need or may be provided to a medical facility or practitioner overseeing medical care of the individual in need. In particular embodiments, the cells are obtained from a human subject that is from 1 day old to 30 days old. For example, the human subject may be, or may be no more than, 1; 2; 3; 4; 5; 6; 7; 8; 9; 10; 11; 12; 13; 14; 15; 16; 17; 18; 19; 20; 21; 22; 23; 24; 25; 26; 27; 28; 29; or 30 days old. The human subject may also be less than one day old.

Embodiments of the disclosure encompass immortalized neonatal CSCs that are derived from cells from a myocardium of a mammal, including a human. In particular embodiments, human neonatal heart CSCs may have a specific genotype and/or phenotype. The immortalized human neonatal heart CSCs may be provided to an individual in need thereof following determination of a certain genotype or phenotype of the cells that is suitable for the intended function of the cells upon therapeutic use, in certain embodiments. However, in some preferred embodiments, a conditioned medium produced from culturing the immortalized human neonatal heart CSCs is provided to an individual in need thereof following determination of a certain genotype or phenotype of the cells that is suitable for producing conditioned media having the intended function. In specific embodiments, the immortalized human neonatal heart CSCs are CD117+ cells, and in particular embodiments the immortalized human neonatal heart CSCs are CD117+, CD90+, CD105+, CD73+, CD44+, CD47+ and CD31-, CD45-. In other embodiments, the immortalized human neonatal heart CSCs also have one, two, three, four or five or more of the following characteristics: GATA4-, CD44+, CD31⁻, tryptase-, CD80-, CD45-, CD86-, HLA Class I+, and HLA Class II-. In other embodiments, the immortalized human neonatal heart CSCs also have one, two, three, four or five or more of the following characteristics: GATA4-, CD44+, CD73+, CD47+ CD31-, tryptase-, CD80-, CD45-, CD86-, HLA Class I+, and HLA Class II-.

In some embodiments, the immortalized human neonatal heart CSCs naturally secrete one or more proteins or factors that are beneficial for repair, regeneration or remodeling of localized cells or tissues. In some embodiments, the present disclosure provides conditioned medium produced by culturing the immortalized human neonatal heart CSCs under conditions whereby they secrete one or more proteins or factors that are beneficial for repair, regeneration or remodeling of localized cells or tissue. Such proteins or factors may be of any kind, but in specific embodiments they are cytokines, pro-angiogenic factors, growth factors, transcription factors, miRNAs and so forth. In some embodiments, the cells secrete one or more of, or any combination of, VEGF-A, HGF, SCF, SDF-1$\alpha$, ANG-1, bFGF, PDGFB, and IGF-1. In some embodiments, the cells secrete factors selected from one or more of, or any combination of, VEGF-A, HGF, SCF, SDF-1$\alpha$, ANG-1, bFGF, PDGFB, and IGF-1, and in some embodiments the present disclosure provides conditioned medium comprising one or more of such factors. In certain embodiments, the cells are manipulated to increase secretion of SDF-1$\alpha$, VEGF-A, PGDF-A and/or FGF-2, or a combination thereof. Such manipulation may be by any manner but in specific embodiments the manipulation encompasses cell engineering by recombinant technology for increased expression of one or more of these factors and/or other factors not expressed by these cells but of therapeutic value. Thus, in some embodiments, conditioned medium from such genetically engineered cells is provided by the present disclosure. Another manipulation contemplated for use in the present invention is the exposure of the immortalized human neonatal heart CSCs to heat shock factors and/or other agent that increase the secretion of the cytokines.

In some embodiments, the immortalized human neonatal heart CSCs naturally express one or more proteins or factors that are beneficial for repair, regeneration or remodeling of localized cells or tissue, either directly or indirectly. For example, the immortalized human neonatal heart CSCs may express VEGF-A and/or SDF-1$\alpha$. In addition, similar to human neonatal heart CSCs, the immortalized human neonatal heart CSCs may have activation of expression of HSF-1, HSP60, and/or HSP70, either naturally or by manipulation of the cells to increase expression of HSF-1, HSP60, and/or HSP70 (Sharma et al, 2017). Accordingly, the present disclosure provides in some embodiments conditioned medium produced by such cells, said conditioned medium comprising VEGF-A and/or SDF-1$\alpha$. In some embodiments, the present disclosure provides conditioned medium produced by such cells comprises expression of HSF-1, HSP60, and/or HSP70 naturally or by manipulation. In some embodiments, the conditioned medium (CM) of immortalized neonatal cardiac stem cells comprises a unique combination of paracrine factors (e.g., cytokines and growth factors) within a pre-specified range as detailed in Table 1.

Further, analysis of the exosome content in the secretome of immortalized neonatal cardiac stem cells indicated that the number of exosomes with a diameter of 137.6nM (mode value, SD=49.1nM) is within the range of 1.1e+9 $\pm$ 3.81e+7 particles/ml and that these exosomes display the surface markers CD63, CD73+, CD47+ and they are negative for CD31 and CD45 (see, e.g., Figure 8).

The immortalized human neonatal heart CSCs may be present as a plurality of cells, and the plurality may be 100% homogeneous with respect to the desired cardiac stem cells. Or, in some embodiments, the plurality may have less than 100% homogeneity, such as having 99, 98, 97, 96, 95, 94, 93, 92, 91, 90, 85, 80, 75, 70, 65, 60, 55, 50, 45, 40, 35, 30, or 25% homogeneity, or having at least that percentage of homogeneity, with respect to the desired immortalized human neonatal heart CSCs . The immortalized human neonatal heart CSCs may be utilized in methods when they are present in a plurality have 100% homogeneity or having less than 100% homogeneity, such as having 99, 98, 97, 96, 95, 94, 93, 92, 91, 90, 85, 80, 75, 70, 65, 60, 55, 50, 45, 40, 35, 30, or 25% homogeneity.

The immortalized human neonatal heart CSCs may be stored for a period of time before their use (e.g., use in culture for producing a conditioned media disclosed herein), including under suitable medium, temperature, and oxygen level conditions, or they may be used without significant storage time. In specific embodiments, the storage cell medium comprises one or more heat shock response inducers.

The immortalized human neonatal heart CSCs may be used for allogeneic therapies as they express low levels of MHC Class II, or co-stimulator proteins CD88 and CD80. These cells do not initiate an immune response when transplanted into another patient's immune system. In such a case, the cells may be used as an off the shelf product for clinical applications.

In particular embodiments, CSCs from a young individual have demonstrated strong reparative and/or regenerative abilities when compared to similarly derived cells from adult hearts (Sharma et al., 2017). These increased abilities are due in part to a more potent secretome by the young cells, in specific embodiments. This implies that the young cells may be a preferable allogenic product, in at least specific embodiments. Moreover, in some embodiments, these superior reparative and/or regenerative properties are maintained after immortalization of the human neonatal heart CSCs according to the present disclosure. Thus, for example, in some embodiments, secretomes produced by immortalized human neonatal heart CSCs are superior to secretomes produced by CSCs derived from adult heart cells for use in inducing repair and/or regeneration of damaged cardiac tissue. Accordingly, the present disclosure provides methods of inducing repair and/or regeneration of damages to cardiac tissue comprising administering to a patient in need thereof a composition comprising conditioned media from one or more immortalized human neonatal heart CSCs. In some embodiments, a population of human neonatal heart CSCs is resuspended in a composition comprising conditioned media from one or more immortalized human neonatal heart CSCs and the composition comprising the conditioned media and the resuspended human neonatal heart CSCs is administered to the patient.

## II. **Methods for Isolation of nCSCs**

The present disclosure also provides a novel method for isolating CD117+ CSCs from neonatal tissue without the need to expose the collected cells to a substrate comprising a surface with a moiety that binds CD117 (i.e., c-kit). This method is in contrast to prior art methods for isolating CD117+ hCSCs, which all comprise exposing the collected cells to such a substrate (e.g., a plate, bead, or column) comprising a CD117-binding moiety (such as an antibody) in order to select out the CD117+ cells from the heterogeneous population of cells present in the sample.

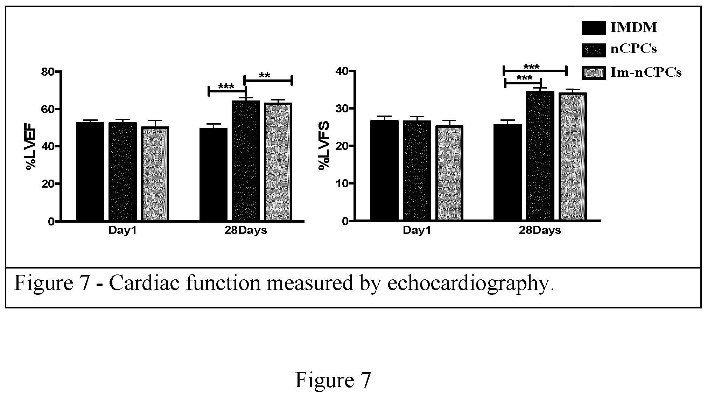

The methods disclosed herein are based in part on the surprising discovery that, in contrast to adult human heart, the neonatal human heart contains a homogenous population of cardiogenic CD117+ stem cells with very little or no contamination of hematopoietic and endothelial progenitor cells. Further, we have discovered that the CD117+ cells in neonatal human heart give rise to clones that can proliferate in culture, even when initially seeded as single cells, and their proliferation rates relate to the level of CD117 positivity of the cells. Thus, cells with high-degrees of CD117+ expression generally proliferate faster than cells with lower expression of CD117, but all cells that we observed proliferating expressed CD117 to at least some degree. Moreover, CD117+ cells in the neonatal cardiac tissue display no evidence of senescence and expand into large cell numbers with significant cardiac repair and regeneration activities when tested in animal models of myocardial infarction (see. e.g., **Figure** 7).

Thus, based at least in part on these surprising discoveries, the present disclosure provides a method for isolating and propagating human neonatal heart CSCs (e.g., homogenous populations of CD117+ neonatal heart CSCs), directly from neonatal heart tissue with no prior selection with CD117 magnetic beads (or any other CD117+ cell enrichment procedure). This method provides for selection of a unique cell population defined by the phenotype CD90$^+$, CD105$^+$, CD117$^+$, CD44$^+$, CD73$^+$, CD47$^+$, CD31$^-$, CD34$^-$, CD45$^-$ and tryptase negative that has not been disclosed prior to this disclosure. In addition, such methods provide significant advantages over prior art methods, as they provide for a single cell donor and donor independence in obtaining large quantities of the cells, ensure cell homogeneity, and avoid regulatory concerns with exposing cells intended for therapy to non GMP quality materials (i.e. the CD117 magnetic beads). Further, they reduce the level of processing that must occur between obtaining a neonatal cardiac sample and providing a population CD117+ cells, e.g., for therapeutic purposes, for producing conditioned media and/or for immortalization according to the methods disclosed herein.

In various embodiments, the present disclosure also provides a method for isolating CD117+ CSCs from neonatal cardiac tissue, the method comprising isolating or having isolated one or more clonal isolate cells from a neonatal cardiac tissue, and culturing the one or more cells in a suitable culture medium to promote proliferation, wherein the isolating does not comprise contacting the cells with a moiety that binds CD117 (e.g., a CD117 antibody). In some embodiments, the method comprises initiating a culture or a plurality of cultures, each culture with one or more cell from the cardiac tissue. In some embodiments, each culture or each of the plurality of cultures is initially seeded with only one cell from the cardiac tissue. This may be accomplished by any suitable method known in the art, e.g., by FACS sorting or limited dilution culturing of cells obtained from a neonatal cardiac tissue sample.

The method may comprise monitoring the proliferation speed of the culture or of one or more of the plurality of cultures. The method may comprise approximating the level of CD117 positivity of the culture or of one or more of the plurality of cultures by comparing the proliferation speed of the culture or cultures with the proliferation speed of a reference sample or reference value of a known CD117+ expression. Clones that proliferate more rapidly than other clones may be chosen for further expansion, characterization, and/or cryopreservation. Clones that express higher levels of CD117 positivity than other clones may be chosen for further expansion, characterization, and/or cryopreservation. Further characterization may include, without limitation, performance of secretome analysis (e.g., by ELISA, MSD or the like), such as, e.g., analysis for human VEGFA, SDF-1$\alpha$, PDGFB, IGF-1, ANG-1, bFGF, SCF and/or HGF; phenotypic characterization (e.g., by flow cytometry), such as, e.g., analysis for cell surface expression of markers (e.g., Mesenchymal stem cell markers CD105 or CD90, stem cell marker CD117, endothelial cell marker CD31, mast cell marker tryptase, and/or hematopoietic cell lineage marker CD45); senescence analysis; and/or functional characterization, e.g., ability to promote angiogenesis, resistance to oxidative stress, and/or therapeutic efficacy, e.g., in *in vivo* cardiac injury models. In some embodiments, such methods may further comprise immortalization of a cell isolated using methods known in the art or disclosed herein. The immortalization may be by any suitable means. In some embodiments, the immortalization is by expression of telomerase. Illustratively, the telomerase may be encoded by a human TERT (hTERT) gene. The human neonatal heart CSCs may be transfected with hTERT, infected with a recombinant virus capable of transferring the hTERT gene into the cell, or delivered to the CSCs using any suitable methodology known and available in the art.

In some embodiments, the present disclosure provides a method for approximating the level of CD117 positivity in a culture of neonatal CSCs or in a plurality of cultures of neonatal CSCs, the method comprising determining the speed of proliferation of the cells in the culture and comparing that speed to the speed of proliferation of a reference sample or reference value of a known CD117+ expression.

In some embodiments, such methods of isolating CD117+ neonatal heart CSCs (as disclosed herein) are utilized for therapeutic purposes, e.g., such as the therapeutic purposes disclosed in US Pat. Pub. No. US2015/0328263, incorporated herein by reference in its entirety, including without limitation, the administration of CD1 17+ or otherwise immortalized CSCs to a subject in need thereof; administering conditioned medium from the immortalized CSCs to a subject in need thereof; and/or administering a combination of the immortalized CSCs and conditioned medium from the immortalized CSCs to a subject in need thereof. Such therapeutic purposes typically include administering any of the above composition to the subject in need thereof after immortalization of the neonatal CSCs according to a method known in the art or disclosed herein.

One of skill in the art recognizes that in addition to the above methods for isolating CD117+ CSCs from neonatal cardiac tissue, there are also other routine methods for isolating the desired nCSCs, and isolation may occur by any suitable means. For example, traditional methods for isolating CD117+ adult CSCs comprise exposing the collected cells to a substrate comprising a surface with a moiety that binds CD117, such as a CD117 antibody, in order to pull out the CD117+ cells from the population of cells present in the sample, In some embodiments the present disclosure does not utilize such methods.

One of skill in the art also recognizes that there are routine methods to obtain cells from a human myocardium and further process the cells thereafter. In particular embodiments, there are methods of isolating the desired human neonatal heart CSCs by obtaining tissue from a human myocardium (such as obtained from the right atrial appendage or "RAA" of the heart), including from a neonatal myocardium, such as by biopsy. The myocardium may be from an individual with no known cardiac abnormalities. The myocardium may be from an individual with end stage heart failure or from an individual with congenital heart disease, and in these cases the myocardium may or may not be normal. The extracted tissue may be exposed to particular medium while separating singular cells from their tissue, including by cutting the tissue, such as in the presence of collagenase. In specific embodiments, the tissue and tissue fragments are allowed to sediment while in a medium, and the supernatant is obtained. Cells may be collected from the supernatant and suspended in culture medium for a suitable period of time. Following this, the desired CD117+ human neonatal heart CSCs, for example, are isolated therefrom. The isolation of the desired human neonatal heart CSCs may occur by any means disclosed herein or known in the art. In some embodiments, the isolation is via a method disclosed herein that does not comprise exposing the collected cells to a substrate comprising a surface with a moiety that binds CD117, such as a CD117 antibody.

In certain embodiments, once the desired human neonatal heart CSCs are isolated, they may be cultured under standard conditions, including suitable passaging. The medium for culture of the cells may or may not be substantially identical to the medium employed when the cells are delivered to an individual. The medium may or may not comprise one or more heat shock response inducers.

## III. Methods of Use of Cells of the Disclosure and the Conditioned Media and Secretomes Thereof

Methods of the disclosure include use of certain nCSCs (and, in certain specific embodiments, CD117+ mesenchymal-like cells), or conditioned medium produced by culturing such cells, for therapy for at least one medical condition in an individual in need thereof. In some embodiments, the cells are isolated via a method disclosed herein, wherein the isolation does not comprise exposing the collected cells to a substrate comprising a surface with a moiety that binds CD117, such as a CD117 antibody, or that binds any other surface protein. In some embodiments, the clonal cell isolates are immortalized (e.g., via hTERT immortalization). In specific embodiments, the cells, conditioned medium produced by culturing the cells or the cells resuspended in their conditioned medium are useful for medical conditions wherein creating an anti-inflammatory or anti-fibrotic effect and/or facilitating tissue repair, regeneration, and/or replacement would be therapeutically useful.

In particular embodiments, the medical condition is a cardiac medical condition. In specific embodiments, a therapeutically effective amount of immortalized human neonatal heart CSCs, conditioned medium from the immortalized human neonatal heart CSCs , or cells resuspended in conditioned medium from the immortalized human neonatal heart CSCs described herein is provided to the individual, and in particular embodiments the conditioned medium is delivered to the individual locally to the area requiring treatment via catheter, direct injection or, in some cases, by topical administration. In other embodiments, a therapeutically effective amount of immortalized human neonatal heart CSCs, conditioned medium from the immortalized human neonatal heart CSCs, or cells resuspended in conditioned medium from the immortalized human neonatal heart CSCs described herein is provided to the individual intravenously. The individual receiving the therapy may be of any gender or age. The individual may or may not be diagnosed by a medical practitioner with the cardiac medical condition. In certain embodiments, the individual has a personal or family medical history of a cardiac medical condition. The individual may be at risk for a cardiac medical condition, such as smoking, high low density lipoprotein (LDL) plasma levels and/or low high density lipoprotein (HDL) plasma levels, uncontrolled hypertension, obesity (more than 20% over one's ideal body weight), uncontrolled diabetes, high C-reactive protein plasma levels, or a combination thereof. In specific embodiments an individual is provided one or more methods of the disclosure upon diagnosis of a certain genotype or phenotype for the individual.

Upon isolation of the desired cells from the source individual, e.g., via a method disclosed herein, wherein the isolation does not comprise exposing the collected cells to a substrate comprising a surface with a moiety that binds CD117 (i.e., c-kit), such as a CD117 antibody, or a moiety that binds another cell surface protein, and prior to their delivery to the individual in need thereof or to the delivery of conditioned medium from the cells to the individual, the cells may be further modified, such as through manipulation with genetic engineering for recombinant expression of one or more expression constructs, further culturing and/or enrichment, and so forth. Such further modifications may also comprise immortalization of the cells as described herein. Such practices are routine in the art. An expression construct transfected into the cells may be of any kind, but in specific embodiments the construct expresses a cytokine, pro-angiogenic factor, growth factor, transcription factor, and so forth. In specific embodiments the construct expresses VEGF-A, HGF, SCF, SDF-1$\alpha$, ANG-1, HSF-1, PGDF-A, FGF-2, or a combination thereof and/or expresses another protein that directly or indirectly increases their expression. The cells may be exposed to one or more agents to increase secretion and/or expression of certain proteins.

In certain embodiments, a therapeutically effective amount of cells as described herein are utilized in one or more treatment methods, although in some preferred embodiments the individual instead receives therapeutically effective amounts of conditioned media from the cells; part of all of the secretome from the cells; one or more secreted proteins or other factors (such as exosomes, extracellular vehicles, miRNAs, etc.) from the cells; or a combination thereof. In some embodiments, conditioned media from the cells is administered to the individual in combination with one or more human neonatal heart CSCs. The use of any one of these components or combinations facilitates proliferation of endogenous cardiomyocytes upon their use, in specific embodiments. In certain embodiments, the use of any one of these components or combinations allow for repairing, regenerating or remodeling of the myocardium. In certain embodiments, the use of any one of these components or combinations facilitates or enhances the proliferative, reparative, regenerative or remodeling capacity of endogenous CSCs in the treated the individual.

In particular embodiments, a therapeutically effective amount of the cells, conditioned medium produced by culturing the cells (or immortalized human neonatal heart CSCs) or the cells resuspended in their conditioned medium may be administered more than one time over a period of hours, days, weeks or months. The therapeutically effective dose may be increased or decreased over the course of successive administrations.

In particular embodiments, when cells are provided to the individual, they may be provided in conjunction with (in the same or a different composition) or at the same time as another therapeutic moiety. That is, in specific embodiments the cells are administered to the individual at substantially the same time as one or more agents that enhance the function of the cells upon their administration *in vivo.* Such an agent may be of any kind, but in specific embodiments the agent is one or more heat shock response inducers.

Although in some cases one or more agents are provided to the individual at substantially the same time as the cells, in specific embodiments the cells are exposed to one or more agents prior to their delivery to the individual, wherein the agent(s) enhances the function of the cells upon *in vivo* delivery. In specific embodiments, the cells are exposed to one or more agents while in culture. The cells may be exposed to the agents one or multiple times, and when the cells are passaged in culture, the agents may or may not be present in subsequent media. In specific embodiments, the agents enhance therapeutic use of the cells by increasing expression of one or more genes, increasing secretion of one or more proteins or other factors (such as miRNAs), or a combination thereof. In particular embodiments, the genes or proteins are cytokines, pro-angiogenic factors, growth factors, transcription factors, miRNAs, and exosomes (small carrying vehicles that contain concentrated proteins and miRNAs), and so forth. In certain embodiments, the agents are heat shock response inducers.

In one embodiment, a particular and therapeutically effective number of the cells will be provided to the individual. For example, in some embodiments, less than 100 million, e.g., from 1-40 million or 1-50 million cells are provided to the individual. In specific embodiments, 1-20 million cells are provided, but in some embodiments the number of millions of cells is 1-19, 1-18, 1-17, 1-16, 1-15, 1-14, 1-13, 1-12, 1-11, 1-10, 1-9, 1-8, 1-7, 1-6, 1-5, 1-4, 1-3, 1-2, 2-20, 2-19, 2-18, 2-17, 2-16, 2-15, 2-14, 2-13, 2-12, 2-11, 2-10, 2-9, 2-8, 2-7, 2-6, 2-5, 2-4, 2-3, 3-20, 3-19, 3-18, 3-17, 3-16, 3-15, 3-14, 3-13, 3-12, 3-11, 3-10, 3-9, 3-8, 3-7, 3-6, 3-5, 3-4, 4-20, 4-19, 4-18, 4-17, 4-16, 4-15, 4-14, 4-13, 4-12, 4-11, 4-10, 4-9, 4-8, 4-7, 4-6, 4-5, 5-20, 5-19, 5-18, 5-17, 5-16, 5-15, 5-14, 5-13, 5-12, 5-11, 5-10, 5-9, 5-8, 5-7, 5-6, 6-20, 6-19, 6-18, 6-17, 6-16, 6-15, 6-14, 6-13, 6-12, 6-11, 6-10, 6-9, 6-8, 6-7, 7-20, 7-19, 7-18, 7-17, 7-16, 7-15, 7-14, 7-13, 7-12, 7-11, 7-10, 7-9, 7-8, 8-20, 8-19, 8-18, 8-17, 8-16, 8-15, 8-14, 8-13, 8-12, 8-11, 8-10, 8-9, 9-20, 9-19, 9-18, 9-17, 9-16, 9-15, 9-14, 9-13, 9-12, 9-11, 9-10, 10-20, 10-19, 10-18, 10-17, 10-16, 10-15, 10-14, 10-13, 10-12, 10-11, 11-20, 11-19, 11-18, 11-17, 11-16, 11-15, 11-14, 11-13, 11-12, 12-20, 12-19, 12-18, 12-17, 12-16, 12-15, 12-14, 12-13, 13-20, 13-19, 13-18, 13-17, 13-16, 13-15, 13-14, 14-20, 14-19, 14-18, 14-17, 14-16, 14-15, 15-20, 15-19, 15-18, 15-17, 15-16, 16-20, 16-19, 16-18, 16-17, 17-20, 17-19, 17-18, 18-20, 18-19, or 19-20, for example.

In particular embodiments, one can isolate the proteins, miRNAs, or any other factors within the conditioned media made by culturing the immortalized human neonatal heart CSCs disclosed herein. In specific embodiments, from the conditioned media the secretome itself or one or more components of the secretome, such as proteins and exosomes, are provided to an individual. Exosomes have very strong reparative and/or regenerative abilities by themselves and may be provided therapeutically to the individual rather than the cells. In specific embodiments, the conditioned media is provided therapeutically to the individual rather than the cells.

### IV. Cardiac Medical Conditions

Embodiments of the disclosure concern treatment of one or more cardiac medical conditions with conditioned media made from culturing immortalized neonatal CSCs described herein and/or with neonatal heart CSCs isolated via a method disclosed herein. Particular aspects for such embodiments result in reversal of one or more cardiac medical conditions or improvement of at least one symptom of one or more cardiac medical conditions. In exemplary embodiments, the cardiac medical condition is heart failure. The heart failure may be the result of one or more causes, including coronary artery disease, heart attack, high blood pressure, faulty heart valves, cardiomyopathy (such as caused by disease, infection, alcohol abuse and the toxic effect of drugs, such as cocaine or some drugs used for chemotherapy), idiopathic cardiomyopathy, congenital heart disease and/or genetic factors.

Particular but exemplary indications of embodiments of the disclosure include at least applications for heart failure, including congestive heart failure; prevention of ventricular remodeling; and/or cardiomyopathy. Other indications may also include coronary artery disease, ischemic heart disease, valvular heart disease, stroke secondary to structural heart interventions, etc. In specific embodiments, methods and compositions of the disclosure provide myocardial repair and/or regeneration that is sufficient to treat, including reverse, an established cardiac medical condition such a cardiomyopathy or congestive heart failure.

In cases where the individual has heart failure, the patient may have preserved ejection fraction (EF) or reduced ejection fraction (EF). Ejection fraction is characterized by microvascular rarefaction and extensive myocardial fibrosis both of which may be improved with a therapeutically effective amount of cells, conditioned medium from the immortalized human neonatal heart CSCs , or cells resuspended in conditioned medium from the immortalized human neonatal heart CSCs as previous studies using an isoproterenol-induced cardiomyopathy model showed that CSCs improves cardiac function in rodent models (Sharma et al; 2017).

In cases where the individual has cardiomyopathy, the cardiomyopathy may be ischemic or non-ischemic cardiomyopathy. The cardiomyopathy may be caused by long-term high blood pressure, heart valve problems, heart tissue damage from a previous heart attack, chronic rapid heart rate, metabolic disorders, nutritional deficiencies, pregnancy, alcohol abuse, drug abuse, chemotherapy drugs, viral infection, hemochromatosis, genetic condition, elevated cholesterol levels, or a combination thereof. Cardiomyopathy may also have no identified cause, i.e. idiopathic cardiomyopathy.

A therapeutically effective amount or number of cells, conditioned medium from the immortalized human neonatal heart CSCs, or cells resuspended in conditioned medium from the immortalized human neonatal heart CSCs may prevent chemotherapy induced cardiotoxicity. The disclosed invention may also be used for extended organ preservation during transport in organ replacement procedures.

### V. Inflammatory Diseases

Embodiments of the disclosure provide compositions comprising neonatal heart CSCs, immortalized neonatal heart CSCs, conditioned medium from the neonatal heart CSCs, or the immortalized neonatal heart CSCs, and neonatal heart CSCs, resuspended in conditioned medium from the c-neonatal heart CSCs or the immortalized human neonatal CSCs, each of which have anti-inflammatory and anti-apoptotic properties and can be used to treat one or more diseases where a primary component of the disease process involves an inflammatory process. Non-limiting examples of such diseases that involve an inflammatory component and may be treated with such compositions of the present disclosure include kidney disorders (both acute kidney injury and chronic kidney disease), ischemic stroke, arthritis, dry eyes, neurodegenerative diseases including Parkinson's disease, critical limb ischemia, COVID-19, and other inflammatory diseases. Accordingly, in some embodiments, the present disclosure provides a composition comprising neonatal heart CSCs, immortalized neonatal heart CSCs, conditioned medium from the neonatal heart CSCs or the immortalized neonatal heart CSCs, and neonatal heart CSCs resuspended in conditioned medium from the neonatal heart CSCs or the immortalized neonatal CSCs for use in treating an inflammatory disease. In some embodiments, the present disclosure provides a composition comprising neonatal heart CSCs , immortalized neonatal heart CSCs , conditioned medium from the neonatal heart CSCs or the immortalized neonatal heart CSCs and neonatal heart CSCs resuspended in conditioned medium from the neonatal heart CSCs or the immortalized neonatal CSCs for use in treating a disease selected from a kidney disorders (both acute kidney injury and chronic kidney disease), ischemic stroke, arthritis, dry eyes, a neurodegenerative diseases, Parkinson's disease, and critical limb ischemia. In some embodiments, the present disclosure provides a method of treating an inflammatory disease comprising administering to a subject in need thereof an effective dose of a composition comprising neonatal heart CSCs, immortalized neonatal heart CSCs, conditioned medium from the neonatal heart CSCs or the immortalized neonatal heart CSCs, and neonatal heart CSCs re-suspended in conditioned medium from the neonatal heart CSCs or the immortalized neonatal CSCs. In some embodiments, the present disclosure provides a method of treating a kidney disorders (both acute kidney injury and chronic kidney disease), ischemic stroke, arthritis, dry eyes, a neurodegenerative diseases, Parkinson's disease, or critical limb ischemia comprising administering to a subject in need thereof an effective dose of a composition comprising neonatal heart CSCs, immortalized neonatal heart CSCs, conditioned medium from the neonatal heart CSCs or the immortalized neonatal heart CSCs , and neonatal heart CSCs re-suspended in conditioned medium from the neonatal heart CSCs or the immortalized neonatal CSCs.

## VI. Wound Healing

Fibrosis and inflammation are two important pathways in wound healing. Accordingly, some embodiments of the disclosure provide compositions comprising neonatal heart CSCs, immortalized neonatal heart CSCs, conditioned medium from the neonatal heart CSCs or the immortalized neonatal heart CSCs, and neonatal heart CSCs resuspended in conditioned medium from the neonatal heart CSCs or the immortalized neonatal CSCs, each of which can be used to treat wounds and to facilitate wound healing. In particular embodiments, one can isolate the proteins, miRNAs, or any other factors within the conditioned media made by culturing the immortalized neonatal CSCs disclosed herein, and such isolated proteins, miRNAs or factors may be administered to a subject with a wound to treat the wound. In specific embodiments, the immortalized neonatal CSCs , the conditioned media, the secretome itself or one or more components of the secretome, such as proteins and exosomes, responsible for regulating tissue fibrosis and cell function such as proliferation, migration and matrix synthesis can be used to advance the progression and resolution of wounds.

## VII. Combination Therapy

In some embodiments, an individual who has received therapy of the disclosure, or is receiving, or will receive therapy of the disclosure, is also provided another therapy for the targeted medical condition. For example, in some embodiments, an individual who has received therapy of the disclosure to treat a cardiac medical condition, or is receiving, or will receive therapy of the disclosure to treat a cardiac medical condition, is also provided another therapy for a cardiac medical condition. The therapy of the present disclosure may precede or follow the other treatment. The therapy of the present disclosure may precede or follow the other treatment by intervals ranging from minutes to hours to days to weeks or months. In embodiments where the other agent and the instant therapy are given separately to the individual, one would generally ensure that a significant period of time did not expire between the time of each delivery, such that the therapy of the disclosure and the additional therapy would still be able to exert an advantageously combined effect on the individual. In such instances, it is contemplated that one may contact the individual with both modalities simultaneously or within minutes of each other or within about 1-12, 6-12, or 12-24 h of each other, for example. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In specific embodiments, the therapy of the present disclosure and the additional therapy are provided at the same time. In specific embodiments, the therapy of the present disclosure and the additional therapy are provided at different times. The separate entities may be within the same compositions or they may be comprised in separate compositions. In cases wherein the therapy of the present disclosure and the second therapy are provided at different times, they may be separated by any suitable range in times, such as minutes, hours, days, weeks, or months. In embodiments wherein they are provided separately, the order of delivery of two (or more) therapies may be of any suitable order, including delivery of cells, secretomes, and/or conditioned media prior to, concurrent with or subsequent to another therapy.

Examples of other treatments to be employed with the therapy of the disclosure include one or more of the following: ACE Inhibitors, Aldosterone Inhibitor, Angiotensin II Receptor Blocker (ARBs); Beta-Blockers, Calcium Channel Blockers, Cholesterol-Lowering Drugs, Digoxin, Diuretics, Inotropic Therapy, Potassium or Magnesium, Vasodilators, anticoagulant medication, aspirin, surgery, VAD implantation, VAT, coronary bypass, percutaneous coronary intervention (PCI) or a combination thereof.

## VIII. Kits of the Disclosure

Any of the immortalized neonatal heart CSCs described herein, or conditioned media from such CSCs may be comprised in a kit. The kit may additionally comprise other agents for therapy of a cardiac medical condition.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a vial. The kits of the present disclosure also will typically include a means for containing the one or more compositions in close confinement for commercial sale. Such containers may include injection or blow-molded plastic containers into which the desired vials are retained. In specific embodiments the cells are delivered in a frozen state and may or may not be provided in plastic vials.

The composition may be formulated into a syringe able composition. In which case, the container means may itself be a syringe, pipette, and/or other such like apparatus, from which the formulation may be applied to an affected area of the body, injected into an animal, and/or even applied to and/or mixed with the other components of the kit. However, the components of the kit may be provided as dried powder(s). When reagents and/or components are provided as a dry powder, the powder can be reconstituted by the addition of a suitable solvent. It is envisioned that the solvent may also be provided in another container means.

The kits of the present disclosure will also typically include a means for containing the vials in close confinement for commercial sale, such as, e.g., injection and/or blow-molded plastic containers into which the desired vials are retained.

In particular embodiments, the kit comprises reagents and/or tools for determining that an individual has a cardiac medical condition. In some embodiments, the kit comprises one or more additional therapies for a cardiac-related medical condition, such as one or more of ACE Inhibitor, aldosterone inhibitor, angiotensin II receptor blocker (ARBs); beta-blocker, calcium channel blocker, cholesterol-lowering drug, digoxin, diuretics, inotropic therapy, potassium, magnesium, vasodilator, anticoagulant medication, aspirin, TGF-beta inhibitor, and a combination thereof.

## VII. Pharmaceutical Compositions

Embodiments of pharmaceutical compositions of the present disclosure comprise an effective amount of neonatal heart CSCs or conditioned media from such cells dispersed in a pharmaceutically acceptable carrier. The effective amount of neonatal CSCs may include any suitable number of cells. In some embodiments, the effective amount less than 100 million cells, e.g., from 1-40 million or 1-50 million cells. In some embodiments, the effective amount comprises from 1-20 million cells, but in some embodiments the number of millions of cells is 1-19, 1-18, 1-17, 1-16, 1-15, 1-14, 1-13, 1-12, 1-11, 1-10, 1-9, 1-8, 1-7, 1-6, 1-5, 1-4, 1-3, 1-2, 2-20, 2-19, 2-18, 2-17, 2-16, 2-15, 2-14, 2-13, 2-12, 2-11, 2-10, 2-9, 2-8, 2-7, 2-6, 2-5, 2-4, 2-3, 3-20, 3-19, 3-18, 3-17, 3-16, 3-15, 3-14, 3-13, 3-12, 3-11, 3-10, 3-9, 3-8, 3-7, 3-6, 3-5, 3-4, 4-20, 4-19, 4-18, 4-17, 4-16, 4-15, 4-14, 4-13, 4-12, 4-11, 4-10, 4-9, 4-8, 4-7, 4-6, 4-5, 5-20, 5-19, 5-18, 5-17, 5-16, 5-15, 5-14, 5-13, 5-12, 5-11, 5-10, 5-9, 5-8, 5-7, 5-6, 6-20, 6-19, 6-18, 6-17, 6-16, 6-15, 6-14, 6-13, 6-12, 6-11, 6-10, 6-9, 6-8, 6-7, 7-20, 7-19, 7-18, 7-17, 7-16, 7-15, 7-14, 7-13, 7-12, 7-11, 7-10, 7-9, 7-8, 8-20, 8-19, 8-18, 8-17, 8-16, 8-15, 8-14, 8-13, 8-12, 8-11, 8-10, 8-9, 9-20, 9-19, 9-18, 9-17, 9-16, 9-15, 9-14, 9-13, 9-12, 9-11, 9-10, 10-20, 10-19, 10-18, 10-17, 10-16, 10-15, 10-14, 10-13, 10-12, 10-11, 11-20, 11-19, 11-18, 11-17, 11-16, 11-15, 11-14, 11-13, 11-12, 12-20, 12-19, 12-18, 12-17, 12-16, 12-15, 12-14, 12-13, 13-20, 13-19, 13-18, 13-17, 13-16, 13-15, 13-14, 14-20, 14-19, 14-18, 14-17, 14-16, 14-15, 15-20, 15-19, 15-18, 15-17, 15-16, 16-20, 16-19, 16-18, 16-17, 17-20, 17-19, 17-18, 18-20, 18-19, or 19-20, for example, including any integer number of cells in between. For example, in one particular embodiment, the pharmaceutical compositions of the present disclosure comprise 10 million neonatal heart CSCs dispersed in a pharmaceutically acceptable carrier. In one particular embodiment, the pharmaceutical compositions of the present disclosure comprise 10 million neonatal CD117+ CSCs and conditioned media from cultured neonatal CSCs. In some embodiments, the cultured neonatal heart CSCs from which the conditioned media is harvested are immortalized neonatal human neonatal heart CSCs.

In one particular embodiment, the pharmaceutical compositions of the present disclosure comprise an effective amount of neonatal CSCs (e.g., 10 million cells) and a concentrated preparation of secreted factors present in conditioned media from cultured neonatal CSCs. Such concentrated preparations of secreted factors may be prepared, e.g., by filtering conditioned media from the cells to remove some or all of the media, while retaining all or some of the secreted factors present in the media. Such concentrated factors may be stored in pellet form. Such concentrated factors may be resuspended in a pharmaceutically acceptable carrier, excipients, diluent, surfactant, and/or vehicles for storage or for administration to a subject. The concentrated factors may be resuspended in the pharmaceutically acceptable carrier, excipients, diluent, surfactant, and/or vehicles alone or in combination with a population of neonatal heart CSCs (e.g., an effective dose of the CSCs). In some embodiments, the cultured neonatal CSCs, from which the conditioned media is harvested to prepare the concentrated preparation of secreted factors, are immortalized neonatal CSCs. In some embodiments, the CSCs that are administered to a subject and/or that are included in the pharmaceutical compositions disclosed herein are not immortalized.

The conditioned media may be formulated for administration to a subject. In some embodiments, bioactive factors present in the conditioned media are enriched for (e.g., by filtration of the conditioned media) and the bioactive factors are reformulated in a pharmaceutical composition comprising one or more pharmaceutically acceptable carrier, excipients, diluent, surfactant, and/or vehicles. The phrases "pharmaceutical or pharmacologically acceptable" refers to molecular entities and compositions that do not produce an adverse, allergic or other untoward reaction when administered to an animal, such as, for example, a human, as appropriate. The preparation of a pharmaceutical composition that comprises cells are known to those of skill in the art in light of the present disclosure, as exemplified by Remington: The Science and Practice of Pharmacy, 21st Ed. Lippincott Williams and Wilkins, 2005, incorporated herein by reference. Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art. Except insofar as any conventional carrier is incompatible with the active ingredient, its use in the pharmaceutical compositions is contemplated.

In some embodiments, the present disclosure provides a pharmaceutical composition comprising one or more human neonatal heart CSCs isolated according to a method disclosed herein, said composition further comprising one or more pharmaceutically acceptable carrier, excipients, diluent, surfactant, and/or vehicles. For example, in some embodiments, the composition comprises a plurality of human neonatal heart CSCs isolated according to a method disclosed herein formulated in PlasmaLyte (Baxter, Deerfield, IL). In some embodiments, the human neonatal heart CSCs are immortalized. In some embodiments, the immortalization is via hTERT expression.

In some embodiments, the present disclosure provides a pharmaceutical composition comprising conditioned media from a culture of human neonatal heart CSCs isolated according to a method disclosed herein, said composition further comprising one or more pharmaceutically acceptable carrier, excipients, diluent, surfactant, and/or vehicles. The conditioned media may be formulated for direct administration to a subject. In particular embodiments, the conditioned media is enriched for bioactive factors by filtration and enriched factors are reformulated in a pharmaceutical composition comprising one or more pharmaceutically acceptable carrier, excipients, diluent, surfactant, and/or vehicles. For example, in some embodiments, the composition comprises enriched bioactive factors from a conditioned media from a culture of a plurality of neonatal heart CSCs isolated according to a method disclosed herein formulated in PlasmaLyte (Baxter, Deerfield, IL). In some embodiments, the human neonatal heart CSCs are immortalized. In some embodiments, the immortalization is via hTERT expression.

## EXAMPLES

The following examples are included to demonstrate preferred embodiments of the invention. It should be appreciated by those of skill in the art that the techniques disclosed in the examples that follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1: Isolation of Neonatal CD117-Positive Cardiac Progenitor Cells

Cardiac medical conditions and inflammatory diseases, including heart failure, are in need of effective therapy for patients of any age, including babies, children, and adults. In particular, compositions and methods are needed for regenerating functional myocardium in adults and children with cardiac medical conditions, such as heart failure caused by damaged myocardial tissue, for example.

Described in this Example is a novel method for isolating and immortalizing CD117+ nCSCs that can be used for producing conditioned media, which can be administered to patients in need thereof to treat damaged myocardium.

We have discovered that, in contrast to adult human heart, the neonatal human heart contains a homogenous population of cardiogenic CD117+ stem cells with very little or no contamination of hematopoietic and endothelial progenitor cells. Further, we have discovered that these neonatal human heart derived stem cells display no evidence of senescence, give rise to clones that can proliferate and expanded into large cell numbers with significant cardiac repair and regeneration activities when tested in animal models of myocardial infarction. Accordingly, based in part on these observations, we have determined that we can obtain these neonatal heart stem cells directly from the neonatal heart tissue with no prior selection with CD117 magnetic beads, as has been done in the literature so far. This is a significant advantage as it avoids regulatory concerns with exposing cells intended for therapy to non-GMP quality materials (i.e. the CD117 magnetic beads).

Here we describe the methodology and certain data on the isolation and functional characterization of human neonatal heart stem cell clones obtained without CD117 antibody enrichment.

This study was approved by the Institutional Review Board and the Institute of Animal Care and Use Committee (IACUC) at the School of Medicine, University of Maryland. After parental or patient consent was given, specimens (20±40 mg) from the right atrial appendage (RAA) were obtained from neonate (1day-30 days old) during routine cardiac surgeries.

1. RAA tissue was transferred into a 100 mm Petri dish filled with saline solution to wash it. This step is repeated twice. Forceps sterilized in Steri 250 (Inotech) were used to remove fibrotic tissue and fat from the cardiac specimen. The samples were then transferred into Ham's F12 medium and minced in 1-2 mm$^2$ slices.

2. The tissue fragments were transferred to a 50 ml tube and allowed to sediment. The supernatant was removed and the sedimented pieces were resuspended in 5-10 ml of Collagenase type II, CSL2 (Worthington # 4177). The collagenase was dissolved at a concentration of 1-2 mg/ml in Ham's F12 medium depending on tissue size and type. Subsequently, the samples were incubated on a shaker at 200rpm for 30-45 min at 37°C.

3. Following the collagenase treatment, the tube was removed from the shaker, undigested pieces were allowed to sediment and the supernatant containing the released cells was centrifuged at 1000rpm for 10min at 15°C, resuspended in the growth medium (Ham's F12 nutrient mixture supplemented with 10% FBS, 0.2 mM L-Gluthatione, 10ng/ml bFGF, 0.005 U/ml of EPO) and plated in T25 flasks containing growth medium and the flasks were placed in an incubator at 37 °C, under 5% CO2. After 72hrs, non- adherent cells were removed by aspiration, the adherent cells were washed with PBS, and fresh growth media was added. Once cells reached 90-95% confluency, growth medium was removed and the cells were detached by using 3ml of TrypLE™. After cell detachment, growth medium was added and the cell suspension was transferred to a 50 ml test tube, centrifuged at 1000rpm for 10 mins at 15°C, and the supernatant was discarded to collect the cells pallet and obtain the cell count.

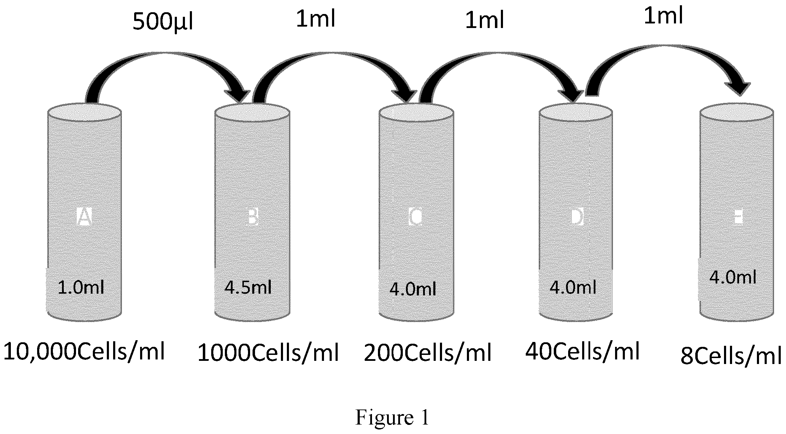

4. As shown in Figure 1, 10,000 cells were used for serial dilution to obtain single cell isolates of the clones. Approximately 8 cells/ml were used to obtain single cell isolates. 50ul from the 8 cells/ml suspension were aliquoted in individual wells in a 96 well plate. Wells with more than one cell were excluded from the experiment through visual inspection under a microscope.

5. Cell numbers were manually counted and population doubling levels (PDLs) were calculated using the formula: PDL = 3.32(log (total cells at harvest/total cells at seed)). Subcultures were carried out until the clones reached senescence and no change in PDL was observed from one subculture to the next.

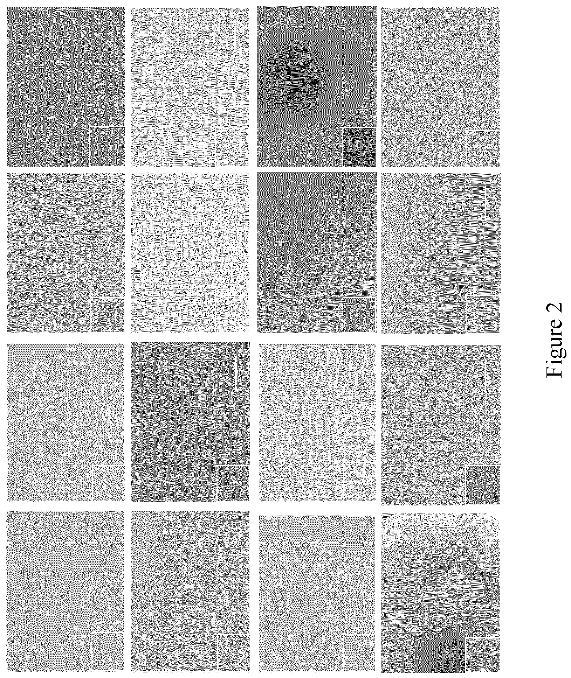

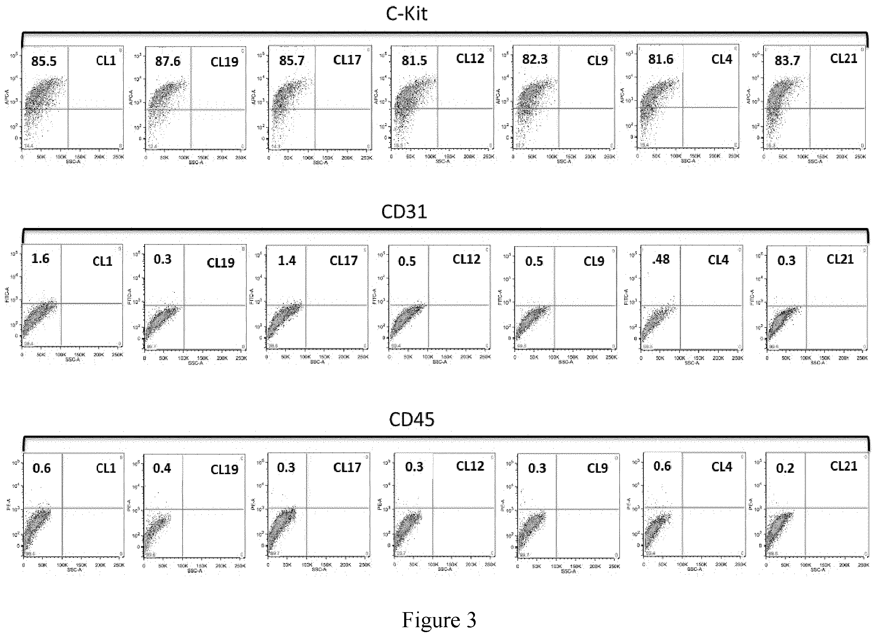

From the total 80 wells, 24 wells were detected with one cell. Out of 24 plated single cells, 18 formed actively proliferating clones (cloning efficiency 75%). Figure 2 shows representative images of some of the single cell cultures.
Proliferating clones were fixed with 4% paraformaldehyde and labeled with fluorochrome-conjugated primary antibodies specific for Mesenchymal stem cell markers CD105 and CD90, stem cell marker CD117, endothelial cell marker CD31, mast cell marker tryptase, hematopoietic cell lineage marker CD45, as well as CD44 and CD47 were evaluated by flow cytometry on a Becton-Dickinson Fortessa with 10,000 events/sample were collected.
FACS analysis indicated that while all 18 proliferating clones were CD117 positive, only 7 were highly (>80%) CD117 positive (Figure 3) while the remaining 11 clones were less positive (<74%).
In addition, these high CD117 positive clones proliferated about 2 times faster than the low CD117 positive clones (data not shown) and were negative for CD31 and CD45.
Taken together these results indicate that, in contrast to CD117+ cells from animals and adult human hearts, human neonatal heart CD117+ cells are surprisingly devoid of contaminating endothelial and hematopoietic progenitor cells, which is not only biologically unique and unexpected but also as is currently done in the literature.

**Example 2: Immortalization of Neonatal CD117-Positive Cardiac Progenitor Cells**

Neonatal CD117+ CSCs secrete proteins that induce repair, regeneration and/or remodeling of the damaged myocardium and improve cardiac function. Immortalization of these CD117+ neonatal CSCs would enable the production of an unlimited supply of conditioned medium comprising such secreted proteins. The present example provides such an immortalization.
Neonatal CD117+ CSC clones are isolated according to the methods disclosed above in Example 1, and clones are expanded in culture. Typically, clones expressing high levels of CD117 were immortalized, however, in some instances clones that express low levels of CD117 were also immortalized.
Immortalization was achieved by transfection of human neonatal CD117+ heart CSC clones with a lentiviral vector expressing hTERT, and clones expressing hTERT were isolated, propagated and stored for long-term use. Figure 4, shows a non-limiting example of a construct for use in the immortalization of neonatal CD117+ human neonatal heart CSCs according to the present invention.

Additionally, reversibly immortalized neonatal CD117+ CSC clones can be constructed by flanking the immortalization gene shown in Figure 4 with a sequence that can be used for gene excision, e.g., according to the methods described in Hu, X., et al., Oncotarget, 2017, Vol. 8, (No. 67), pp: 111847-111865, which is incorporated herein by reference in its entirety. In one example, the immortalization gene is flanked by FRT sites: one 5' to the CMV promoter and one 3' to the Puromycin resistance cassette. FLP recombinase may then be used to facilitate excision of the immortalization cassette, thereby reversing the immortalization. In addition to their utility for production of large quantities of conditioned media, such cells can also be used for direct administration to patients after immortalization is reversed prior to delivery of the cells to the patient, optionally in combination with conditioned media made from the cells.

A kill switch control is optionally incorporated in the vector by, for example, incorporating the gene for Thymidine kinase of herpes simplex virus (HSV-TK). The HSV-TK phosphorylates the prodrug ganciclovir (GCV), an analog of guanosine nucleoside. The phosphorylated GCV is incorporated into host DNA and terminates the elongation of DNA strands, resulting in cell death. Thus, treatment of a patient that has received an administration of such reversibly immortalized neonatal heart CSCs with GCV results in the death of the administered cells expressing HSV-TK, ensuring drug safety. Other kill switches are known in the art and may be used in the constructs disclosed herein.

**Example 3 Characterization of Neonatal CSCs and their Cultured Medium**

**a. Telomeres and Telomere length**

To calculate the length of telomeres of human neonatal heart CSC clones, flow cytometry analysis is performed using fluorescence in situ hybridization and a fluorescein-conjugated PNA probe (Telomere PNA kit/FITC) for flow cytometry from Dako (Cat # K5327)). Cell line 1301, which is tetraploid and has long telomeres (>30 kbp) is used as a control. Relative telomere length (RTL) is calculated by using following formula-

$$RTL= \frac{(mean\ FL1\ sample\ cells\ with\ probe - mean\ FL1\ samples\ cells\ without\ probe\ X\ DNA\ index\ of\ control\ cells X100}{(mean\ FL1\ control cells\ with\ probe - mean\ FL1\ control\ without\ probe\ X\ DNA\ index\ of\ sample\ cells}$$

**b. Senescence-Associated ß-Galactosidase Staining**

Cellular senescence is assessed using a ß-galactosidase staining kit (Cat # 9860, Cell Signaling technology, Boston, MA) following the manufacturer's instructions. Briefly, human neonatal heart CSCs before and after immortalization (5.0 X 104) are be plated in a 24 well. After 24hrs growth medium is removed from the cells, cells are rinsed with PBS and fixed with 1x fixative solution for 15 minutes at room temperature. Cells are incubated overnight with 1 ml of ß-galactosidase staining solution and are imaged the next day. Results demonstrate that these neonatal human heart derived stem cells display no evidence of senescence. **(Figure 5)**

**c. Paracrine Factor Secretion**

Immortalized neonatal CSCs were grown in complete xeno-free media until they reached 85-90% confluence. The cells were washed with warm serum and growth factor free basal Ham's F12 medium twice, after that basal Ham's F12 were added and incubated for 48hours at 37C hours to obtain total conditioning medium (TCM). The TCM was precleared of cellular debris and particulate matter by centrifugation at 1000g for 30 minutes, followed by 20,000g for 30 minutes to remove microvesicles (MVs), then concentrated using 3 KDa filters (Millipore Inc, Billerica, MA). Total protein content was quantified using TCA-NLS method followed by the bicinchoninic assay (BCA) method (Thermofisher, Waltham, MA). Im-nCSCs-derived TCM's appearance is clear, transparent and apoptotic bodies free (Figure 8). IM-nCPCs TCM is dsDNA free 0.2-0.4ng/ml (Permissible limit upto 200ng/ml). TCM is 90-99.0% CD63+ and negative for CD45 and CD31 (Fig).

To normalize the protein content, we used the following formula:

(concentration factor) x (total volume of medium)/total protein content of conditioned medium

The conditioned medium is quantified using TCA-NLS method followed by BCA method and normalized to a total of 1mg protein. 8 paracrine factors, VEGFA, SDF-1$\alpha$, PDGFB, IGF- 1, ANG-1, bFGF, SCF and HGF are analyzed using Meso-Scale Discovery device\, according to the manufacturer's protocol. Results demonstrate that Im-nCSC TCM secrete all 8 paracrine factors at the levels shown in Table 1.

**Table. 1.** Cardioprotective/anti-inflammatory/angiogenenic/anti-apotosis factors in the secretome of immortalized nCSCs

| Factor | Mean (pg/ml) | Sum of CV | Range (pg/ml) |
|--------|--------------|-----------|---------------|
| Ang-1 | 587.1 | 4.3 | 587.1-618.3 |
| bFGF | 7.1 | 4.3 | 7.1-7.4 |
| HGF | 223.6 | 0.3 | 223.6-265.4 |
| IGF-1 | 14.6 | 13.4 | 14.6-31.3 |
| PDGF-B | 2.9 | 10.4 | 2.9-3.9 |
| SCF | 1.2 | 21.3 | 1.2-1.9 |
| SDF-1a | 591.7 | 15.3 | 591.7-736.3 |
| VEGF-A | 433.9 | 5.5 | 353.1-433.9 |

### Example 4 - Functional Activity of Immortalized Neonatal CSCs and their Cultured Medium

#### a. In-Vitro Activity

#### a1. Angiogenesis Activity

To test the pro-angiogenic effects of TCM on blood vessel formation, HMEC cells were subjected to an *in vitro* standard angiogenesis assay using Im-nCPCs derived TCM (Im-nCSCs TCM) and IMDM basal media as negative control with HMECs complete medium as positive control. Briefly, tube assay formation is performed to evaluate the angiogenesis potential of CM. Formation of tube-like structures is assessed in a Matrigel-coated 24 well plate (BD Biosciences, San Jose, CA), as described previously. Briefly, human microvascular endothelial cells (HMEC-1, ATCC® CRL-3243™) are counted and seeded at the density of 20,000 cells/mm2 plated on reduced growth factor containing Matrigel (Product #354230, BD Biosciences, San Jose, CA) with the addition of (i) endothelial complete cell medium (Lonza) as positive control, (ii) Conditioned media from Im-nCSCs, or (iii) basal medium (IMDM) as negative control. Cells are imaged after 6-12 hours and a complete image of each well is reconstructed. The total tube length is then measured using ImageJ64, NIH (http://rsb.info.nih.gov/ij).

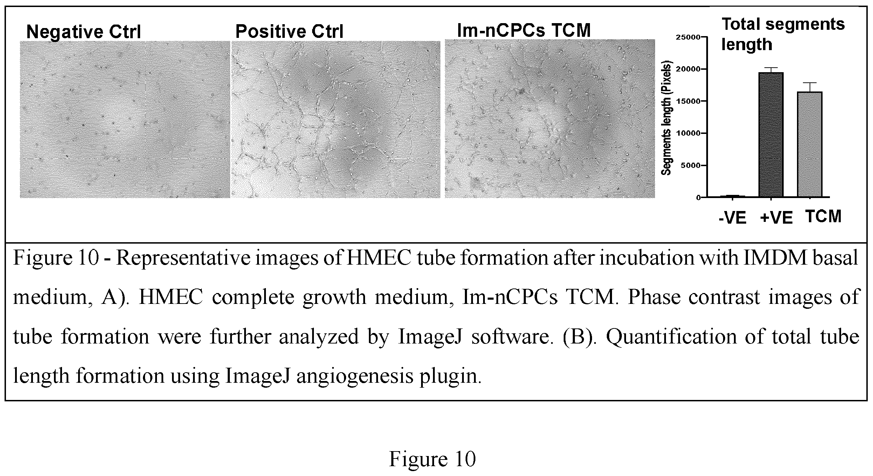

The results demonstrated that the ability of the HMECs to form complex and mature endothelial tube networks was absent in IMDM but in the presence of Im-nCSCs TCM, HMECs formed mature tubes as shown in Figure 10.

#### a2. Wound Healing Activity

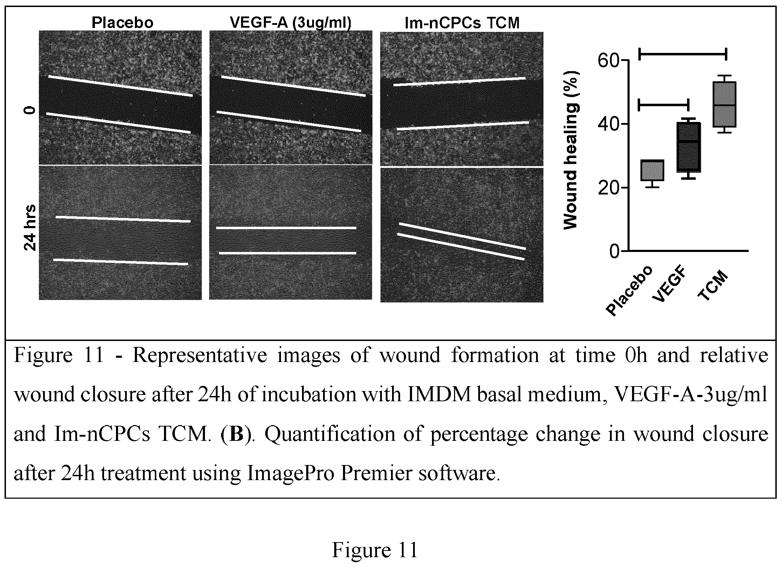

To assess the wound healing potential of Im-nCSCs TCM, in Vitro wound healing assay was performed to assess the relative migratory potential of cells treated with total conditioning medium. HMECs (HMEC-1 ATCC® CRL-3243™) were seeded in a 12 well plate to create a confluent monolayer. After 12 hours of serum starvation with basal medium, a 1mL pipette tip was used to create linear scratches along the cell monolayer to simulate a wound. Cell debris was removed by washing the cells once with basal medium. Cells were treated with total conditioning medium derived from Im-nCSCs, PBS as a negative control and VEGF-A (3.0ug/ul) as positive control. Images of each wound were taken at specific reference points along the scratch at times 0h and 22h after treatment. HMECs were stained with Calcein AM cell-permeant dye (ThermoFisher Scientific, Inc.) and imaged pre and post-treatment with conditioned media obtained from Im-nCSCs TCM. ImagePro software was used to measure the total wound area before and after treatment to calculate percentage change in wound closure. The results showed that Im-nCSCs derived TCM significantly increased the wound healing process as compared to the negative control (Figure 11).

#### a3. Protection Against H2O2 Induced Cell Apoptosis

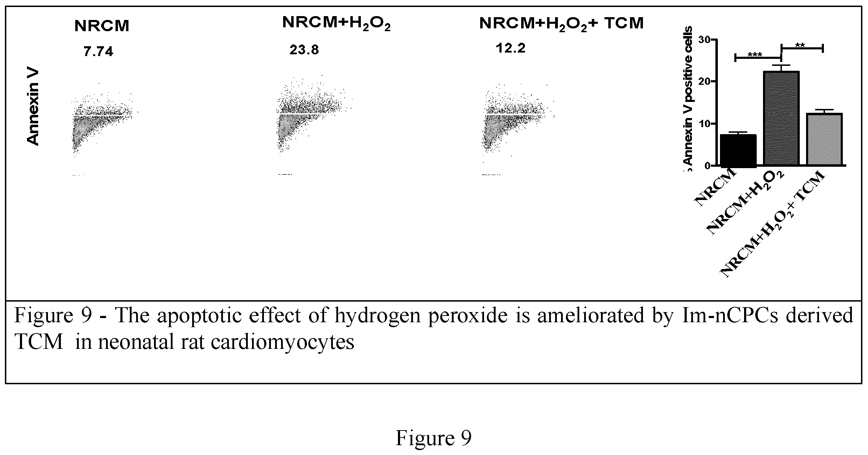

Assessment of apoptosis in response to oxidative stress is performed using the Annexin V apoptosis detection kit (Cat # 556547, BD pharmingin). Briefly, neonatal rat cardiomyocytes (NRCMs) were purchased from Lonza Walkersville, Inc (RCM-561)) and cultured according to manufacturer's instructions. Briefly, all the components of the rat cardiomyocyte growth medium (RCGM) were thawed overnight in the cold room and mixed. 10 wells of 24 well plates were coated with nitrocellulose/methanol mix (0.1 cm2 nitrocellulose dissolved in 1.0 ml of methanol) and each vial of rat cardiomyocytes was suspended in 10 ml of complete RCGM. I ml of the cell suspension was transferred to each well (3x10$^5$ cells/well) after overnight incubation NRCM were treated with 100 $\mu$M hydrogen peroxide in the absence or presence of Im-nCSC-derived

conditioned media (50 $\mu$M) for 6 hr in serum-free basal media (n = 4 technical replicates) followed by flow analysis for annexin V/PI using the BD Pharmingen FITC annexin V Apoptosis Detection Kit I (Cat# 556547). Data showed that Im-nCSCs TCM significantly decreased the expression of early apoptosis marker annexin V (Figure 9). These results indicate that Im-nCSCs derived TCM decreased oxidative stress-induced apoptosis.

**b. In-Vivo Activity**

**b1. Cell Transplantation in the Rat Myocardial Infarction (MI) Model**

Myocardial infarction is induced by permanent ligation of the left anterior descending (LAD) coronary artery in immunodeficient male rats (weight, 250-300 g). The heart is exposed via a left thoracotomy, and the proximal LAD is ligated. Afterward, 1 million of nCSCs and Im-nCSCs cells suspended in 100$\mu$L of vehicle (IMDM) are injected into the myocardium at four adjacent sites to the infarct and 100 $\mu$L of vehicle (IMDM) as a control. Baseline echocardiograms are acquired at 1 day before myocardial infarct surgery. Echocardiographic examinations are also performed at 7 days and at 28 days post-myocardial infarction. Two-dimensional and M-mode echocardiography is performed using the VisualSonics Vevo 2100 ultrasound unit (VisualSonics, Toronto, Canada, www.visualsonics.com)) and to assess fractional area change (FAC). Images are obtained from the parasternal long axis and the parasternal short axis at the mid-papillary level. Myocardial viability is assessed as follows. To calculate infarct size, Masson Trichrome-stained sections at various levels along the long axis are analyzed for collagen deposition. The midline technique for infarct size determination is used as described previously. The stained sections are analyzed by ImagePro software. Briefly, the infarct size is calculated using Masson's trichrome stained sections at various levels along the long axis. To calculate viable and the non-viable tissue, the number of red pixels (viable tissue) and blue pixels (non-viable tissue) is calculated and the ratio of non-viable tissue/overall number of the pixels is presented. Data showed that both nCSCs and nCSCs derived immortalized clones are functional as shown in Fig by significant increase in ejection fraction and fractional shorting after transplantation of nCSCs or nCSCs derived immortalized clones. These results suggest that immortalization did not affect the functional potential of nCSCs derived clones (Figure 7).

**b2. Functional Activity of Im-nCPC Derived TCM in Rat MI Model**

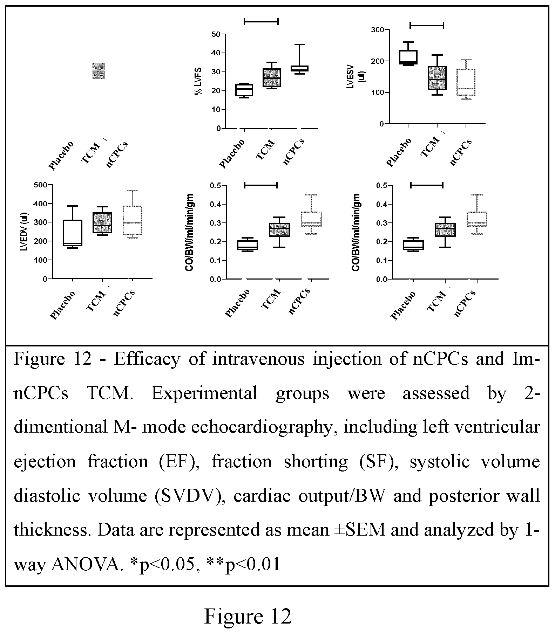

To determine the functional potential of Im-nCSCs derived total conditioned medium (TCM), the rats are subjected to an anteroseptal MI by LAD ligation with a suture. Echocardiography was performed on rats while they were anesthetized and in a supine position, and an ultrasound probe was placed directly on the chest wall. Prior to MI, left ventricular ejection fraction (LVEF) was about 80%. After About ten minutes after MI, the treatments (nCSCs, Im-nCSCs derived TCM and IMDM) was administered intravenously using the tail vein. After 24 hours, heart function was assessed with a baseline echocardiography. Five days later, the treatment group was administered another dose of treatments. nCSCs and Im-nCSCs derived TCM injected animals showed no significant deterioration of LV function from 2 days post-MI to 4 weeks post-MI. At 4 weeks, LVEF was significantly higher in the nCSC-treated group than in the placebo group (Figure 12). Other parameters, including fractional shortening (FS) and decreased end-systolic volume (ESV), were also significantly improved when compared with the placebo group, and other LV functional parameter, including cardiac output/body weight and posterior wall thickness, trended towards improvement and a normal remodeled heart. The nMSC benefit remarkably persisted during the 4-week endpoint. (Figure 12)

**Equivalents**

While the present invention has been described in conjunction with the specific embodiments set forth above, many alternatives, modifications and other variations thereof will be apparent to those of ordinary skill in the art. All such alternatives, modifications and variations are intended to fall within the spirit and scope of the present invention. All of the U.S. patents, U.S. patent application publications, U.S. patent application, foreign patents, foreign patent application and non-patent publications referred to in this specification and/or listed in the Application Data Sheet are incorporated herein by reference, in their entirety. Aspects of the embodiments can be modified, if necessary to employ concepts of the various patents, application and publications to provide yet further embodiments. These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

**REFERENCES**

Roger V L. Epidemiology of heart failure. Circ Res. 2013;113:646-659

Go A S, Mozaffarian D, Roger V L, Benjamin E J, Berry J D, Blaha M J, Dai S, Ford E S, Fox C S, Franco S, Fullerton H J, Gillespie C, Hailpem S M, Heit J A, Howard V J, Huffman M D, Judd S E, Kissela B M, Kittner S J, Lackland D T, Lichtman J H, Lisabeth L D, Mackey R H, Magid D J, Marcus G M, Marelli A, Matchar D B, McGuire D K, Mohler E R, 3rd, Moy C S, Mussolino M E, Neumar R W, Nichol G, Pandey D K, Paynter N P, Reeves M J, Sorlie P D, Stein J, Towfighi A, Turan T N, Virani S, Wong N D, Woo D, Turner M B. Heart disease and stroke statistics-2014 update: A report from the american heart association. Circulation. 2014;129:e28-e292

Rossano J W, Kim J, Decker J A, Price J F, Zafar F, Graves D E, Morales D L, Heinle J S, Bozkurt B, Towbin J A, Denfield S W, Dreyer W J, Jefferies J L. Prevalence, morbidity, and mortality of heart failure-related hospitalizations in children in the united states: A population-based study. Journal of cardiac failure. 2012;18:459-470

Porrello E R, Mahmoud A I, Simpson E, Hill J A, Richardson J A, Olson E N, Sadek H A. Transient regenerative potential of the neonatal mouse heart. Science. 2011;331:1078-1080

Messina E, De Angelis L, Frati G, Morrone S, Chimenti S, Fiordaliso F, Salio M, Battaglia M, Latronico M V, Coletta M, Vivarelli E, Frati L, Cossu G, Giacomello A. Isolation and expansion of adult cardiac stem cells from human and murine heart. Circulation research. 2004;95:911-921

Beltrami A P, Barlucchi L, Torella D, Baker M, Limana F, Chimenti S, Kasahara H, Rota M, Musso E, Urbanek K, Leri A, Kajstura J, Nadal-Ginard B, Anversa P. Adult cardiac stem cells are multipotent and support myocardial regeneration. Cell. 2003;114:763-776

Smith R, Barile L, Cho H C, Leppo M K, Hare J M, Messina E, Giacomello A, Abraham M R, Marban E. Regenerative potential of cardiosphere-derived cells expanded from percutaneous endomyocardial biopsy specimens. Circulation. 2007;115:896-908

Matsuura K, Nagai T, Nishigaki N, Oyama T, Nishi J, Wada H, Sano M, Toko H, Akazawa H, Sato T, Nakaya H, Kasanuki H, Komuro I. Adult cardiac sca-1-positive cells differentiate into beating cardiomyocytes. The Journal of biological chemistry. 2004;279:11384-11391

He J Q, Vu D M, Hunt G, Chugh A, Bhatnagar A, Bolli R. Human cardiac stem cells isolated from atrial appendages stably express c-kit. PloS one. 2011;6:e27719

Stolzing A, Sethe S, Scutt A M. Stressed stem cells: Temperature response in aged mesenchymal stem cells. Stem cells and development. 2006;15:478-487

Mishra R, Vijayan K, Colletti E J, Harrington D A, Matthiesen T S, Simpson D, Goh S K, Walker B L, Almeida-Porada G, Wang D, Backer C L, Dudley S C, Jr., Wold L E, Kaushal S. Characterization and functionality of cardiac progenitor cells in congenital heart patients. Circulation. 2011;123:364-373

Sharma , Mishra R, Bigham E. G, Wehman B, Khan M, Xu H, Saha P, Goo Ah. G, Datla R. S, Chen L, Tulapurkar E. M, Taylor S. B, Yang P, Karathanasis S, Goodlett R D, Kaushal S. A Deep Proteome Analysis Identifies the Complete Secretome as the Functional Unit of Human Cardiac Progenitor Cells. Circulation Res. 2017;120:816-834

Bu L, Jiang X, Martin-Puig S, Caron L, Zhu S, Shao Y, Roberts D J, Huang P L, Domian I J, Chien K R. Human isl1 heart progenitors generate diverse multipotent cardiovascular cell lineages. Nature. 2009;460:113-117

Bolli R, Chugh A R, D'Amario D, Loughran J H, Stoddard M F, Ikram S, Beache G M, Wagner S G, Leri A, Hosoda T, Sanada F, Elmore J B, Goichberg P, Cappetta D, Solankhi N K, Fahsah I, Rokosh D G, Slaughter M S, Kajstura J, Anversa P. Cardiac stem cells in patients with ischaemic cardiomyopathy (scipio): Initial results of a randomised phase 1 trial. Lancet. 2011;378:1847-1857

Makkar R, Smith R, Cheng K, Malliaras K, Thomson L E, Berman D, Czer L S, Marban L, Mendizabal A, Johnston P V, Russell S D, Schuleri K H, Lardo A C, Gerstenblith G, Marban E. Intracoronary cardiosphere-derived cells for heart regeneration after myocardial infarction (caduceus): A prospective, randomised phase 1 trial. Lancet. 2012;379:895-904

Simpson D L, Mishra R, Sharma S, Goh S K, Deshmukh S, Kaushal S. A strong regenerative ability of cardiac stem cells derived from neonatal hearts. Circulation. 2012;126:S46-53

Kajstura J, Leri A, Finato N, Di Loreto C, Beltrami C A, Anversa P. Myocyte proliferation in end-stage cardiac failure in humans. Proceedings of the National Academy of Sciences of the United States of America. 1998;95:8801-8805

Anversa P, Kajstura J. Ventricular myocytes are not terminally differentiated in the adult mammalian heart. Circulation research. 1998;83:1-14

Rajabi M, Kassiotis C, Razeghi P, Taegtmeyer H. Return to the fetal gene program protects the stressed heart: A strong hypothesis. Heart failure reviews. 2007;12:331-343

Malliaras K, Makkar R, Smith R, Cheng K, Wu E, Bonow R O, Marban L, Mendizabal A, Cingolani E, Johnston P V, Gerstenblith G, Schuleri K H, Lardo A C, Marban E. Intracoronary cardiosphere-derived cells after myocardial infarction: Evidence of therapeutic regeneration in the final 1-year results of the caduceus trial (cardiosphere-derived autologous stem cells to reverse ventricular dysfunction). Journal of the American College of Cardiology. 2014;63:110-122

Zaruba M, Soonpaa M, Reuter S, Field L J. Cardiomyogenic potential of c-kit(+)-expressing cells derived from neonatal and adult mouse hearts. Circulation. 2010;121:1992-2000

Simpson D, Liu H, Fan T H, Nerem R, Dudley S C, Jr. A tissue engineering approach to progenitor cell delivery results in significant cell engraftment and improved myocardial remodeling. Stem Cells. 2007;25:2350-2357

Chimenti I, Smith R, Li T S, Gerstenblith G, Messina E, Giacomello A, Marban E. Relative roles of direct regeneration versus paracrine effects of human cardiosphere-derived cells transplanted into infarcted mice. Circ Res. 2010;106:971-980

Zhang X, Wang X, Zhu H, Kranias E G, Tang Y, Peng T, Chang J, Fan G C. Hsp20 functions as a novel cardiokine in promoting angiogenesis via activation of vegfr2. PloS one. 2012;7:e32765

Miyata S, Minobe W, Bristow M R, Leinwand L A. Myosin heavy chain isoform expression in the failing and nonfailing human heart. Circ Res. 2000;86:386-390

Razeghi P, Young M E, Alcorn J L, Moravec C S, Frazier O H, Taegtmeyer H. Metabolic gene expression in fetal and failing human heart. Circulation. 2001;104:2923-2931

Afanasiev S A, Falaleeva L P, Rebrova T U, Suslova T E, Popov S V, Karpov R S. Effect of stress-proteins on survival of bone marrow mesenchymal stem cells after intramyocardial transplantation against the background of postinfarction heart remodeling. Bulletin of experimental biology and medicine. 2008;146:111-115

Genth-Zotz S, Bolger A P, Kalra P R, von Haehling S, Doehner W, Coats A J, Volk H D, Anker S D. Heat shock protein 70 in patients with chronic heart failure: Relation to disease severity and survival. International journal of cardiology. 2004;96:397-401

Wang Y, Chen L, Hagiwara N, Knowlton A. Regulation of heat shock protein 60 and 72 expression in the failing heart. Journal of molecular and cellular cardiology. 2010;48:360-366

Reynolds B A, Weiss S. Generation of neurons and astrocytes from isolated cells of the adult mammalian central nervous system. Science. 1992;255:1707-1710

Rietze R L, Reynolds B A. Neural stem cell isolation and characterization. Methods in enzymology. 2006;419:3-23

Mirotsou M, Jayawardena T M, Schmeckpeper J, Gnecchi M, Dzau V J. Paracrine mechanisms of stem cell reparative and regenerative actions in the heart. Journal of molecular and cellular cardiology. 2011;50:280-289

Angelini A, Castellani C, Ravara B, Franzin C, Pozzobon M, Tavano R, Libera L D, Papini E, Vettor R, De Coppi P, Thiene G, Vescovo G. Stem-cell therapy in an experimental model of pulmonary hypertension and right heart failure: Role of paracrine and neurohormonal milieu in the remodeling process. The Journal of heart and lung transplantation: the official publication of the International Society for Heart Transplantation. 2011;30:1281-1293

McGinley L M, McMahon J, Stocca A, Duffy A, Flynn A, O'Toole D, O'Brien T. Mesenchymal stem cell survival in the infarcted heart is enhanced by lentivirus vector-mediated heat shock protein 27 expression. Human gene therapy. 2013;24:840-851

Feng Y, Huang W, Meng W, Jegga A G, Wang Y, Cai W, Kim H W, Pasha Z, Wen Z, Rao F, Modi R M, Yu X, Ashraf M. Heat shock improves sca-1+ stem cell survival and directs ischemic cardiomyocytes toward a prosurvival phenotype via exosomal transfer: A critical role for hsfl/mir-34a/hsp70 pathway. Stem cells. 2014;32:462-472

Cesselli D, Beltrami A P, D'Aurizio F, Marcon P, Bergamin N, Toffoletto B, Pandolfi M, Puppato E, Marino L, Signore S, Livi U, Verardo R, Piazza S, Marchionni L, Fiorini C, Schneider C, Hosoda T, Rota M, Kajstura J, Anversa P, Beltrami C A, Leri A. Effects of age and heart failure on human cardiac stem cell function. The American journal of pathology. 2011;179:349-366

Laflamme M A, Chen K Y, Naumova A V, Muskheli V, Fugate J A, Dupras S K, Reinecke H, Xu C, Hassanipour M, Police S, O'Sullivan C, Collins L, Chen Y, Minami E, Gill E A, Ueno S, Yuan C, Gold J, Murry C E. Cardiomyocytes derived from human embryonic stem cells in pro-survival factors enhance function of infarcted rat hearts. Nature biotechnology. 2007;25:1015-1024

Kannaiyan R, Shanmugam M K, Sethi G. Molecular targets of celastrol derived from thunder of god vine: Potential role in the treatment of inflammatory disorders and cancer. Cancer letters. 2011;303:9-20

Salminen A, Lehtonen M, Paimela T, Kaarniranta K. Celastrol: Molecular targets of thunder god vine. Biochemical and biophysical research communications. 2010;394:439-442

Go A S, Mozaffarian D, Roger V L, Benjamin E J, Berry J D, Blaha M J, et al. Executive summary: heart disease and stroke statistics-2014 update: a report from the american heart association. Circulation. 2014;129(3):399-410. Epub Jan. 22, 2014. doi: 10.1161/01.cir.0000442015.53336.12. PubMed PMID: 24446411.

Go A S, Mozaffarian D, Roger V L, Benjamin E J, Berry J D, Blaha M J, et al. Heart disease and stroke statistics-2014 update: a report from the american heart association. Circulation. 2014;129(3):e28-e292. Epub Dec. 20, 2013. doi: 10.1161/01.cir.0000441139.02102.80. PubMed PMID: 24352519.

Garbern J C, Lee R T. Cardiac stem cell therapy and the promise of heart regeneration. Cell stem cell. 2013;12(6):689-98. Epub Jun. 12, 2013. doi: 10.1016/j.stem.2013.05.008. PubMed PMID: 23746978; PubMed Central PMCID: PMC3756309.

Bolli R, Chugh A R, D'Amario D, Loughran J H, Stoddard M F, Ikram S, et al. Cardiac stem cells in patients with ischaemic cardiomyopathy (SCIPIO): initial results of a randomised phase 1 trial. Lancet. 2011;378(9806):1847-57. Epub Nov. 18, 2011. doi: 10.1016/S0140-6736(11)61590-0. PubMed PMID: 22088800; PubMed Central PMCID: PMC3614010.

Bolli R, Tang X L, Sanganalmath S K, Rimoldi O, Mosna F, Abdel-Latif A, et al. Intracoronary delivery of autologous cardiac stem cells improves cardiac function in a porcine model of chronic ischemic cardiomyopathy. Circulation. 2013;128(2):122-31. Epub Jun. 13, 2013. doi: 10.1161/CIRCULATIONAHA.112.001075. PubMed PMID: 23757309; PubMed Central PMCID: PMC3807652.

Tang X L, Rokosh G, Sanganalmath S K, Yuan F, Sato H, Mu J, et al. Intracoronary administration of cardiac progenitor cells alleviates left ventricular dysfunction in rats with a 30-day-old infarction. Circulation. 2010;121(2):293-305. Epub Jan. 6, 2010. doi: 10.1161/CIRCULATIONAHA.109.871905. PubMed PMID: 20048209; PubMed Central PMCID: PMC2814341.

EP 4 552 690 A2

Beltrami A P, Barlucchi L, Torella D, Baker M, Limana F, Chimenti S, et al. Adult cardiac stem cells are multipotent and support myocardial regeneration. Cell. 2003;114(6):763-76. Epub Sep. 9, 2003. PubMed PMID: 14505575.

Chugh A R, Beache G M, Loughran R I, Mewton N, Elmore J B, Kajstura J, et al. Administration of cardiac stem cells in patients with ischemic cardiomyopathy: the SCIPIO trial: surgical aspects and interim analysis of myocardial function and viability by magnetic resonance. Circulation. 2012;126(11 Suppl 1):S54-64. Epub Sep. 9, 2012. doi: 10.1161/CIRCULATIONAHA.112.092627. PubMed PMID: 22965994; PubMed Central PMCID: PMC3448934.

Pagani F D, DerSimonian H, Zawadzka A, Wetzel K, Edge A S, Jacoby D B, et al. Autologous skeletal myoblasts transplanted to ischemia-damaged myocardium in humans. Histological analysis of cell survival and differentiation. Journal of the American College of Cardiology. 2003;41(5):879-88. Epub Dec. 3, 2003. PubMed PMID: 12628737.

Bergmann O, Bhardwaj R D, Bernard S, Zdunek S, Barnabe-Heider F, Walsh S, et al. Evidence for cardiomyocyte renewal in humans. Science. 2009;324(5923):98-102. Epub Apr. 4, 2009. doi: 10.1126/science.1164680. PubMed PMID: 19342590; PubMed Central PMCID: PMC2991140.

Kajstura J, Rota M, Cappetta D, Ogorek B, Arranto C, Bai Y, et al. Cardiomyogenesis in the aging and failing human heart. Circulation. 2012;126(15):1869-81. Epub Sep. 8, 2012. doi: 10.1161/CIRCULATIONAHA.112.118380. PubMed PMID: 22955965; PubMed Central PMCID: PMC3477474

Ongstad L. E, Mishra R, Belkhodja M, Sharma S, Gaddipati R, Phipps S, Kaushal S, Karathanasis S. Immortalization of Human Neonatal C-kit+ Cardiac Progenitor Cells Preserves Their Phenotype and Function at High Passage. Circulation 2019

Buja, M. Cardiac repair and the putative role of stem cells. Journal of Molecular and Cellular Cardiology 128 (2019) 96-104.

Vujic, A., et al., Molecular mechanisms of heart regeneration. Semin Cell Dev Biol. 2019 Oct 3. pii: S1084-9521(19) 30170-3.

Vincinanza, C, et al., Adult cardiac stem cells are multipotent and robustly myogenic: c-kit expression is necessary but not sufficient for their identification. Cell Death Differ. 2017 Dec;24(12):2101-2116.

Lewis-McDougall FC, et al., Aged-senescent cells contribute to impaired heart Regeneration. Aging Cell. 2019 Jun;18(3):e12931.

The invention also provides the following numbered embodiments:

1. A composition, comprising a conditioned medium from one or more immortalized neonatal CD117+ cardiac stem cells.

2. The composition of embodiment 1, wherein the cell has one or more of the following characteristics: CD90+, CD105+, CD31-, CD34-, CD45- and/or tryptase negative; and a pharmaceutically acceptable carrier.

3. The composition of any of embodiments 1 or 2, wherein the cell has one or more of the following characteristics: CD44+, CD47+ and/or CD73+.

4. The composition of any one of embodiments 1-3, wherein the cell has one or more the following characteristics: GATA4, CD80-, CD86- and/or Lin-.

5. The composition of embodiment 1, wherein, prior to immortalization, the neonatal CD117+ cardiac cells were isolated from the heart of a neonatal individual who was less than 30 day old.

6. The composition of embodiment 5, wherein the isolation does not comprise contacting the cells with an antibody.

7. The composition of embodiment 5, wherein the isolation does not comprise a cell enrichment step using antibody-based selection.

8. The composition of embodiment 1, wherein the cells are isolated by limited dilution culture.

9. The composition of embodiment 1, wherein the immortalization is achieved by the exogenous expression of hTERT.

10. A composition, comprising immortalized human neonatal CD117+ cardiac stem cells.

11. The composition of embodiment 10, wherein the cell has one or more of the following characteristics: CD90+, CD105+, CD31-, CD34-, CD45- and/or tryptase negative; and a pharmaceutically acceptable carrier.

12. The composition of any of embodiments 10-11, wherein the cell has one or more of the following characteristics: CD44+, CD47+ and/or CD73+.

13. The composition of any one of embodiments 10-12, wherein the cell has one or more the following characteristics: GATA4, CD80-, CD86- and/or Lin-.

14. The composition of embodiment 10, wherein, prior to immortalization, the neonatal CD117+ cardiac cells were isolated from the heart of a neonatal individual who was less than 30 day old.

15. The composition of embodiment 14, wherein the isolation does not comprise contacting the cells with an antibody.

16. The composition of embodiment 14, wherein the isolation does not comprise a cell enrichment step using antibody-based selection.

17. The composition of any one of embodiment 10, wherein the cells are isolated by limited dilution culture.

18. The composition of embodiment 10, wherein the immortalization is achieved by exogenous expression of hTERT.

19. A method of treating an individual for a cardiac medical condition, comprising the step of providing to the individual a therapeutically effective amount of a composition comprising a conditioned medium from immortalized neonatal CD117+ cardiac stem cells.

20. The method of embodiment 19, wherein the composition is selected from any one of the compositions provided in any one of embodiments 1-9.

21. A method of treating an individual for a cardiac medical condition, comprising the step of providing to the individual a therapeutically effective amount of a composition comprising a conditioned medium from immortalized neonatal CD117+ cardiac stem cells in combination with a composition comprising a plurality of neonatal CD117+ cardiac stem cells.

22. The method of embodiment 21, wherein the composition comprising the conditioned medium is selected from any one of the compositions provided in any one of embodiments 1-9.

23. The method of embodiments 21 or 22, wherein the composition comprising a plurality of neonatal CD117+ cardiac stem cells is selected from any one of the compositions provided in any one of embodiments 10-18.

24. A method for repairing or remodeling myocardial tissue in a subject in need thereof comprising contacting the myocardial tissue of the subject with a composition provided in any one of embodiments 1-9, a composition provided in any one of embodiments 10-18, or a combination thereof.

25. A method for isolating a CD117+ stem cell from neonatal cardiac tissue, the method comprising isolating or having isolated one or more cells from a neonatal cardiac tissue and culturing the one or more cells in a suitable culture medium to promote proliferation, wherein the isolating does not comprise contacting the cells with a moiety that binds CD117.

26. The composition of embodiment 25, wherein the isolation does not comprise contacting the cells with an antibody.

27. The method of embodiment 25, wherein the isolation does not comprise contacting the cells with a CD117 antibody.

28. The method of embodiment 25, wherein the isolation is achieved by direct cloning without the need for negative selection against endothelial and/or hematopoietic cells.

**Claims**

1. An immortalized, human, neonatal cardiac stem cell (nCSC), wherein the immortalized, human nCSC is:

   (a) positive for protein expression of CD90, CD105, CD117, CD44, CD73, and CD47, and
   (b) negative for protein expression of CD31, CD34, CD45, and tryptase.

2. The immortalized, human nCSC of claim 1, for use in wound healing and/or treating a cardiac medical condition in a subject in need thereof.

3. The immortalized, human nCSC of claim 1 or 2, for use in wound healing.

4. The immortalized, human nCSC of claim 1 or 2, for use in treating a cardiac medical condition in a subject in need thereof.

5. The immortalized, human nCSC of claim 6, wherein the cardiac medical condition is heart failure, cardiomyopathy, myocardial infarction, or congenital heart disease.

6. The immortalized, human nCSC of claim 1 or 2, which secrete exosomes which are CD63+, CD73+, CD47+, CD45-, and CD31-.

7. The immortalized, human nCSC of claim 1 or 2, for use in producing a conditioned medium comprising IGF-1, ANG-1, HGF, SDF-1α, VEGF-A, bFGF, PDGF-B, and SCF in an *in vitro* culture.

8. A pharmaceutical composition comprising a conditioned medium and a pharmaceutically acceptable carrier, wherein the conditioned medium comprises IGF-1, ANG-1, HGF, SDF-1α, VEGF-A, bFGF, PDGF-B, and SCF.

Figure 1

Figure 2

Figure 3

Figure 4

***Note: Features may not be to scale, please refer to sequence or text map for detailed information.

Figure 5 - Morphology of nCPCs and Im-nCPCs

Figure 5

EP 4 552 690 A2

Figure 6 - Phenotypic Characterization of Im-nCPCs.

Figure 6

Figure 7 - Cardiac function measured by echocardiography.

Figure 7

EP 4 552 690 A2

**S1 S2 S3 CL**

CD 9 — 25kd

Cytochrome C — 15kd

**CD63** — 99.6

**CD45** — 0.0923

**CD31** — 0.161

Figure 8 - Characterization of Im-nCPCs derived total conditioned medium (Im-nCPCs TCM)

Figure 8

Figure 9 - The apoptotic effect of hydrogen peroxide is ameliorated by Im-nCPCs derived TCM in neonatal rat cardiomyocytes

Figure 9

Figure 10 - Representative images of HMEC tube formation after incubation with IMDM basal medium, A). HMEC complete growth medium, Im-nCPCs TCM. Phase contrast images of tube formation were further analyzed by ImageJ software. (B). Quantification of total tube length formation using ImageJ angiogenesis plugin.

Figure 10

Figure 11 - Representative images of wound formation at time 0h and relative wound closure after 24h of incubation with IMDM basal medium, VEGF-A-3ug/ml and Im-nCPCs TCM. (**B**). Quantification of percentage change in wound closure after 24h treatment using ImagePro Premier software.

Figure 11

Figure 12 - Efficacy of intravenous injection of nCPCs and Im-nCPCs TCM. Experimental groups were assessed by 2-dimentional M- mode echocardiography, including left ventricular ejection fraction (EF), fraction shorting (SF), systolic volume diastolic volume (SVDV), cardiac output/BW and posterior wall thickness. Data are represented as mean ±SEM and analyzed by 1-way ANOVA. *p<0.05, **p<0.01

Figure 12

EP 4 552 690 A2

Figure 13 - Normal Karyotype was observed in Im-nCSC. Cytogenetic testing does not detect submicroscopic rearrangement, microdeletion, microduplication, or low level of mosaicism.

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150328263 A

**Non-patent literature cited in the description**

- **KOBAYASHI et al.** *Science*, 2000, vol. 287, 1258-62
- **NAKAMURA et al.** *Transplantation*, 1997, vol. 63 (11), 1541-47
- **WERNER et al.** *Biotechnol Bioeng*, 2000, vol. 68 (1), 59-70
- **AMICONE et al.** *EMBO J.*, 1997, vol. 16 (3), 495-503
- **RAY et al.** *Virology*, 2000, vol. 271, 197-204
- **HAYFLICKET**. *Exp. Cell Res.*, 1961, vol. 25, 585-621
- **GREIDER et al.** *Cell*, 1985, vol. 43, 405-413
- Remington: The Science and Practice of Pharmacy. Lippincott Williams and Wilkins, 2005
- **HU, X. et al.** *Oncotarget*, 2017, vol. 8 (67), 111847-111865
- **ROGER V L**. Epidemiology of heart failure. *Circ Res*, 2013, vol. 113, 646-659
- **GO A S ; MOZAFFARIAN D ; ROGER V L ; BENJAMIN E J ; BERRY J D ; BLAHA M J ; DAI S ; FORD E S ; FOX C S ; FRANCO S**. Heart disease and stroke statistics-2014 update: A report from the american heart association. *Circulation*, 2014, vol. 129, e28-e292
- **ROSSANO J W ; KIM J ; DECKER J A ; PRICE J F ; ZAFAR F ; GRAVES D E ; MORALES D L ; HEINLE J S ; BOZKURT B ; TOWBIN J A**. Prevalence, morbidity, and mortality of heart failure-related hospitalizations in children in the united states: A population-based study. *Journal of cardiac failure*, 2012, vol. 18, 459-470
- **PORRELLO E R ; MAHMOUD A I ; SIMPSON E ; HILL J A ; RICHARDSON J A ; OLSON E N ; SADEK H A**. Transient regenerative potential of the neonatal mouse heart. *Science*, 2011, vol. 331, 1078-1080
- **MESSINA E ; DE ANGELIS L ; FRATI G ; MORRONE S ; CHIMENTI S ; FIORDALISO F ; SALIO M ; BATTAGLIA M ; LATRONICO M V ; COLETTA M**. Isolation and expansion of adult cardiac stem cells from human and murine heart. *Circulation research*, 2004, vol. 95, 911-921
- **BELTRAMI A P ; BARLUCCHI L ; TORELLA D ; BAKER M ; LIMANA F ; CHIMENTI S ; KASAHARA H ; ROTA M ; MUSSO E ; URBANEK K**. Adult cardiac stem cells are multipotent and support myocardial regeneration. *Cell*, 2003, vol. 114, 763-776
- **SMITH R ; BARILE L ; CHO H C ; LEPPO M K ; HARE J M ; MESSINA E ; GIACOMELLO A ; ABRAHAM M R ; MARBAN E**. Regenerative potential of cardiosphere-derived cells expanded from percutaneous endomyocardial biopsy specimens. *Circulation*, 2007, vol. 115, 896-908
- **MATSUURA K ; NAGAI T ; NISHIGAKI N ; OYAMA T ; NISHI J ; WADA H ; SANO M ; TOKO H ; AKAZAWA H ; SATO T**. Adult cardiac sca-1-positive cells differentiate into beating cardiomyocytes. *The Journal of biological chemistry*, 2004, vol. 279, 11384-11391
- **HE J Q ; VU D M ; HUNT G ; CHUGH A ; BHATNAGAR A ; BOLLI R**. Human cardiac stem cells isolated from atrial appendages stably express c-kit. *PloS one*, 2011, vol. 6, e27719
- **STOLZING A ; SETHE S ; SCUTT A M**. Stressed stem cells: Temperature response in aged mesenchymal stem cells. *Stem cells and development*, 2006, vol. 15, 478-487
- **MISHRA R ; VIJAYAN K ; COLLETTI E J ; HARRINGTON D A ; MATTHIESEN T S ; SIMPSON D ; GOH S K ; WALKER B L ; ALMEIDA-PORADA G ; WANG D**. Characterization and functionality of cardiac progenitor cells in congenital heart patients. *Circulation*, 2011, vol. 123, 364-373
- **SHARMA , MISHRA R ; BIGHAM E. G ; WEHMAN B ; KHAN M ; XU H ; SAHA P ; GOO AH. G ; DATLA R. S ; CHEN L ; TULAPURKAR E. M**. Deep Proteome Analysis Identifies the Complete Secretome as the Functional Unit of Human Cardiac Progenitor Cells. *Circulation Res.*, 2017, vol. 120, 816-834
- **BU L ; JIANG X ; MARTIN-PUIG S ; CARON L ; ZHU S ; SHAO Y ; ROBERTS D J ; HUANG P L ; DOMIAN I J ; CHIEN K R**. Human isl1 heart progenitors generate diverse multipotent cardiovascular cell lineages. *Nature*, 2009, vol. 460, 113-117

- **BOLLI R** ; **CHUGH A R** ; **D'AMARIO D** ; **LOUGHRAN J H** ; **STODDARD M F** ; **IKRAM S** ; **BEACHE G M** ; **WAGNER S G** ; **LERI A** ; **HOSODA T**. Cardiac stem cells in patients with ischaemic cardiomyopathy (scipio): Initial results of a randomised phase 1 trial. *Lancet*, 2011, vol. 378, 1847-1857
- **MAKKAR R** ; **SMITH R** ; **CHENG K** ; **MALLIARAS K** ; **THOMSON L E** ; **BERMAN D** ; **CZER L S** ; **MARBAN L** ; **MENDIZABAL A** ; **JOHNSTON P V**. Intracoronary cardiosphere-derived cells for heart regeneration after myocardial infarction (caduceus): A prospective, randomised phase 1 trial. *Lancet*, 2012, vol. 379, 895-904
- **SIMPSON D L** ; **MISHRA R** ; **SHARMA S** ; **GOH S K** ; **DESHMUKH S** ; **KAUSHAL S**. A strong regenerative ability of cardiac stem cells derived from neonatal hearts. *Circulation*, 2012, vol. 126, 46-53
- **KAJSTURA J** ; **LERI A** ; **FINATO N** ; **DI LORETO C** ; **BELTRAMI C A** ; **ANVERSA P**. Myocyte proliferation in end-stage cardiac failure in humans. *Proceedings of the National Academy of Sciences of the United States of America*, 1998, vol. 95, 8801-8805
- **ANVERSA P** ; **KAJSTURA J**. Ventricular myocytes are not terminally differentiated in the adult mammalian heart. *Circulation research*, 1998, vol. 83, 1-14
- **RAJABI M** ; **KASSIOTIS C** ; **RAZEGHI P** ; **TAEGT-MEYER H**. Return to the fetal gene program protects the stressed heart: A strong hypothesis. *Heart failure reviews*, 2007, vol. 12, 331-343
- **MALLIARAS K** ; **MAKKAR R** ; **SMITH R** ; **CHENG K** ; **WU E** ; **BONOW R O** ; **MARBAN L** ; **MENDIZABAL A** ; **CINGOLANI E** ; **JOHNSTON P V**. Intracoronary cardiosphere-derived cells after myocardial infarction: Evidence of therapeutic regeneration in the final 1-year results of the caduceus trial (cardiosphere-derived autologous stem cells to reverse ventricular dysfunction). *Journal of the American College of Cardiology*, 2014, vol. 63, 110-112
- **ZARUBA M** ; **SOONPAA M** ; **REUTER S** ; **FIELD L J**. Cardiomyogenic potential of c-kit(+)-expressing cells derived from neonatal and adult mouse hearts. *Circulation*, 2010, vol. 121, 1992-2000
- **SIMPSON D** ; **LIU H** ; **FAN T H** ; **NEREM R** ; **DUDLEY S C, JR.** A tissue engineering approach to progenitor cell delivery results in significant cell engraftment and improved myocardial remodeling. *Stem Cells*, 2007, vol. 25, 2350-2357
- **CHIMENTI I** ; **SMITH R** ; **LI T S** ; **GERSTENBLITH G** ; **MESSINA E** ; **GIACOMELLO A** ; **MARBAN E**. Relative roles of direct regeneration versus paracrine effects of human cardiosphere-derived cells transplanted into infarcted mice. *Circ Res.*, 2010, vol. 106, 971-980
- **ZHANG X** ; **WANG X** ; **ZHU H** ; **KRANIAS E G** ; **TANG Y** ; **PENG T** ; **CHANG J** ; **FAN G C**. Hsp20 functions as a novel cardiokine in promoting angiogenesis via activation of vegfr2. *PloS one*, 2012, vol. 7, e32765
- **MIYATA S** ; **MINOBE W** ; **BRISTOW M R** ; **LEINWAND L A**. Myosin heavy chain isoform expression in the failing and nonfailing human heart. *Circ Res*, 2000, vol. 86, 386-390
- **RAZEGHI P** ; **YOUNG M E** ; **ALCORN J L** ; **MORAVEC C S** ; **FRAZIER O H** ; **TAEGTMEYER H**. Metabolic gene expression in fetal and failing human heart. *Circulation*, 2001, vol. 104, 2923-2931
- **AFANASIEV S A** ; **FALALEEVA L P** ; **REBROVA T U** ; **SUSLOVA T E** ; **POPOV S V** ; **KARPOV R S.** Effect of stress-proteins on survival of bone marrow mesenchymal stem cells after intramyocardial transplantation against the background of postinfarction heart remodeling. *Bulletin of experimental biology and medicine*, 2008, vol. 146, 111-115
- **GENTH-ZOTZ S** ; **BOLGER A P** ; **KALRA P R** ; **VON HAEHLING S** ; **DOEHNER W** ; **COATS A J** ; **VOLK H D** ; **ANKER S D**. Heat shock protein 70 in patients with chronic heart failure: Relation to disease severity and survival. *International journal of cardiology*, 2004, vol. 96, 397-401
- **WANG Y** ; **CHEN L** ; **HAGIWARA N** ; **KNOWLTON A**. Regulation of heat shock protein 60 and 72 expression in the failing heart. *Journal of molecular and cellular cardiology*, 2010, vol. 48, 360-366
- **REYNOLDS B A** ; **WEISS S**. Generation of neurons and astrocytes from isolated cells of the adult mammalian central nervous system. *Science*, 1992, vol. 255, 1707-1710
- **RIETZE R L** ; **REYNOLDS B A**. Neural stem cell isolation and characterization. *Methods in enzymology*, 2006, vol. 419, 3-23
- **MIROTSOU M** ; **JAYAWARDENA T M** ; **SCHMECK-PEPER J** ; **GNECCHI M** ; **DZAU V J**. Paracrine mechanisms of stem cell reparative and regenerative actions in the heart. *Journal of molecular and cellular cardiology*, 2011, vol. 50, 280-289
- **ANGELINI A** ; **CASTELLANI C** ; **RAVARA B** ; **FRANZIN C** ; **POZZOBON M** ; **TAVANO R** ; **LIBERA L D** ; **PAPINI E** ; **VETTOR R** ; **DE COPPI P**. Stem-cell therapy in an experimental model of pulmonary hypertension and right heart failure: Role of paracrine and neurohormonal milieu in the remodeling process. *The Journal of heart and lung transplantation: the official publication of the International Society for Heart Transplantation*, 2011, vol. 30, 1281-1293

- **MCGINLEY L M ; MCMAHON J ; STOCCA A ; DUFFY A ; FLYNN A ; O'TOOLE D ; O'BRIEN T.** Mesenchymal stem cell survival in the infarcted heart is enhanced by lentivirus vector-mediated heat shock protein 27 expression. *Human gene therapy*, 2013, vol. 24, 840-851

- **FENG Y ; HUANG W ; MENG W ; JEGGA A G ; WANG Y ; CAI W ; KIM H W ; PASHA Z ; WEN Z ; RAO F.** Heat shock improves sca-1+ stem cell survival and directs ischemic cardiomyocytes toward a prosurvival phenotype via exosomal transfer: A critical role for hsfl/mir-34a/hsp70 pathway. *Stem cells*, 2014, vol. 32, 462-472

- **CESSELLI D ; BELTRAMI A P ; D'AURIZIO F ; MARCON P ; BERGAMIN N ; TOFFOLETTO B ; PANDOLFI M ; PUPPATO E ; MARINO L ; SIGNORE S.** Effects of age and heart failure on human cardiac stem cell function. *The American journal of pathology*, 2011, vol. 179, 349-366

- **LAFLAMME M A ; CHEN K Y ; NAUMOVA A V ; MUSKHELI V ; FUGATE J A ; DUPRAS S K ; REINECKE H ; XU C ; HASSANIPOUR M ; POLICE S.** Cardiomyocytes derived from human embryonic stem cells in pro-survival factors enhance function of infarcted rat hearts. *Nature biotechnology*, 2007, vol. 25, 1015-1024

- **KANNAIYAN R ; SHANMUGAM M K ; SETHI G.** Molecular targets of celastrol derived from thunder of god vine: Potential role in the treatment of inflammatory disorders and cancer. *Cancer letters*, 2011, vol. 303, 9-20

- **SALMINEN A ; LEHTONEN M ; PAIMELA T ; KAARNIRANTA K.** Celastrol: Molecular targets of thunder god vine. *Biochemical and biophysical research communications*, 2010, vol. 394, 439-442

- **GO A S ; MOZAFFARIAN D ; ROGER V L ; BENJAMIN E J ; BERRY J D ; BLAHA M J et al.** Executive summary: heart disease and stroke statistics-2014 update: a report from the american heart association. *Circulation*, 22 January 2014, vol. 129 (3), 399-410

- **GO A S ; MOZAFFARIAN D ; ROGER V L ; BENJAMIN E J ; BERRY J D ; BLAHA M J et al.** Heart disease and stroke statistics-2014 update: a report from the american heart association. *Circulation*, 20 December 2013, vol. 129 (3), e28-e292

- **GARBERN J C ; LEE R T.** Cardiac stem cell therapy and the promise of heart regeneration. *Cell stem cell*, 12 June 2013, vol. 12 (6), 689-98

- **BOLLI R ; CHUGH A R ; D'AMARIO D ; LOUGHRAN J H ; STODDARD M F ; IKRAM S et al.** Cardiac stem cells in patients with ischaemic cardiomyopathy (SCIPIO): initial results of a randomised phase 1 trial. *Lancet*, 18 November 2011, vol. 378 (9806), 1847-57

- **BOLLI R, TANG X L ; SANGANALMATH S K ; RIMOLDI O ; MOSNA F ; ABDEL-LATIF A et al.** Intracoronary delivery of autologous cardiac stem cells improves cardiac function in a porcine model of chronic ischemic cardiomyopathy. *Circulation*, 13 June 2013, vol. 128 (2), 122-31

- **TANG X L ; ROKOSH G ; SANGANALMATH S K ; YUAN F ; SATO H ; MU J et al.** Intracoronary administration of cardiac progenitor cells alleviates left ventricular dysfunction in rats with a 30-day-old infarction. *Circulation*, 06 January 2010, vol. 121 (2), 293-305

- **BELTRAMI A P ; BARLUCCHI L ; TORELLA D ; BAKER M ; LIMANA F ; CHIMENTI S et al.** Adult cardiac stem cells are multipotent and support myocardial regeneration. *Cell*, 09 September 2003, vol. 114 (6), 763-76

- **CHUGH A R ; BEACHE G M ; LOUGHRAN R I ; MEWTON N ; ELMORE J B ; KAJSTURA J et al.** Administration of cardiac stem cells in patients with ischemic cardiomyopathy: the SCIPIO trial: surgical aspects and interim analysis of myocardial function and viability by magnetic resonance. *Circulation*, 09 September 2012, vol. 126 (11), S54-64

- **PAGANI F D ; DERSIMONIAN H ; ZAWADZKA A ; WETZEL K ; EDGE A S ; JACOBY D B et al.** Autologous skeletal myoblasts transplanted to ischemia-damaged myocardium in humans. Histological analysis of cell survival and differentiation. *Journal of the American College of Cardiology*, 03 December 2003, vol. 41 (5), 879-88

- **BERGMANN O ; BHARDWAJ R D ; BERNARD S ; ZDUNEK S ; BARNABE-HEIDER F ; WALSH S et al.** Evidence for cardiomyocyte renewal in humans. *Science*, 04 April 2009, vol. 324 (5923), 98-102

- **KAJSTURA J ; ROTA M ; CAPPETTA D ; OGOREK B ; ARRANTO C ; BAI Y et al.** Cardiomyogenesis in the aging and failing human heart. *Circulation*, 08 September 2012, vol. 126 (15), 1869-81

- **ONGSTAD L. E ; MISHRA R ; BELKHODJA M ; SHARMA S ; GADDIPATI R ; PHIPPS S ; KAUSHAL S ; KARATHANASIS S.** Immortalization of Human Neonatal C-kit+ Cardiac Progenitor Cells Preserves Their Phenotype and Function at High Passage. *Circulation*, 2019

- **BUJA, M.** Cardiac repair and the putative role of stem cells. *Journal of Molecular and Cellular Cardiology*, 2019, vol. 128, 96-104

- **VUJIC, A. et al.** Molecular mechanisms of heart regeneration. *Semin Cell Dev Biol*, 03 October 2019, vol. S1084-9521 (19), 30170-3

- **VINCINANZA, C et al.** Adult cardiac stem cells are multipotent and robustly myogenic: c-kit expression is necessary but not sufficient for their identification. *Cell Death Differ*, December 2017, vol. 24 (12), 2101-2116

- **LEWIS-MCDOUGALL FC et al.** Aged-senescent cells contribute to impaired heart Regeneration. *Aging Cell*, June 2019, vol. 18 (3), e12931